# EUROPÄISCHE PATENTSCHRIFT

(11) **EP 3 177 918 B1**
(45) Veröffentlichungstag und Bekanntmachung des Hinweises auf die Patenterteilung: **27.06.2018**
(21) Anmeldenummer: 16754418.8
(22) Anmeldetag: 29.07.2016
(51) Int. Cl.: G01N 33/08, A01K 45/00, A01K 43/00, G01N 21/65

(54) **VERFAHREN UND VORRICHTUNG ZUR EINBRINGUNG EINER ÖFFNUNG IN DIE KALKSCHALE IM BEREICH DES STUMPFEN POLS VON BEBRÜTETEN VOGELEIERN MIT EMBRYO**
METHOD AND DEVICE FOR INSERTING AN OPENING INTO THE LIME SHELL IN THE AREA OF THE BLUNT POLE OF INCUBATED BIRD EGGS WITH AN EMBRYO
PROCEDE ET DISPOSITIF D'AMENAGEMENT D'UNE OUVERTURE DANS LA COQUILLE DANS LA ZONE DU COTE PLAT D'OEFS D'OISEAUX COUVES AYANT UN EMBRYON

(30) Priorität: 29.07.2015 EP 15178951
(43) Veröffentlichungstag der Anmeldung: 14.06.2017
(73) Patentinhaber: Agri Advanced Technologies GmbH, 49429 Visbek (DE)
(72) Erfinder: HURLIN, Jörg, 27801 Dötlingen (DE); MEISSNER, Sven, 09618 Brandersbisdorf (DE); FISCHER, Björn, 09212 Limbach-Oberfrohna (DE)
(74) Vertreter: Vossius & Partner Patentanwälte Rechtsanwälte mbB
(86) Internationale Anmeldenummer: PCT/EP2016/068243
(87) Internationale Veröffentlichungsnummer: WO 2017/017277

(56) Entgegenhaltungen:
- WO-A2-2014/021715
- DE-T2- 69 905 493
- FR-A1- 2 912 216
- US-A- 1 740 661
- US-A1- 2007 137 577
- US-A1- 2008 289 578
- US-A1- 2015 136 030

## Beschreibung

Die Erfindung betrifft ein Verfahren und eine Vorrichtung zur Einbringung einer Öffnung in die Kalkschale im Bereich des stumpfen Pols von bebrüteten Vogeleiern mit darin enthaltenem Embryo. Dabei befinden sich innerhalb des Bereiches des stumpfen Pols eine äußere Membran und eine innere Membran, zwischen denen sich eine Luftkammer befindet, und der Embryo liegt angrenzend an die innere Membran beim mit dem spitzen Pol nach unten gelagerten Brutei an.

Die Erfindung betrifft auch ein Verfahren und eine Vorrichtung zur Geschlechtsbestimmung derartiger Embryos unter Verwendung optischer Daten, die durch Bestrahlen des Embryos durch die geöffnete Kalkschale gewonnen werden.

In der industriellen Geflügelproduktion werden Bruteier von Legehennenlinien oder Geflügelmastlinien zu Beginn des Brutprozesses in sogenannten Bruthorden eingelegt, wobei die Eier derart platziert werden, dass der stumpfe Pol des Eies und somit die im Ei befindliche Luftkammer nach oben gerichtet sind. Der etwas spitzere Pol des ovalen Eies ist dabei nach unten gerichtet. Anschließend werden die Bruthorden zunächst in einen Hordenwagen oder in Hordenregalen in den Brutschrank eingebracht. Nach einer zeitlich vorgegebenen Vorbrut werden die Bruteier dann auf sogenannte Schlupfhorden zum Schlüpfen der Küken umgelagert.

Aufgrund der starken Spezialisierung in der Hühnerzucht (Hybridisierung) ist die Geschlechtsbestimmung des sich entwickelnden Kükens von außerordentlicher Bedeutung. Diese erfolgt derzeit unmittelbar nach dem Schlupf manuell bzw. visuell anhand der Kloakenmorphologie, der Federfarbe oder der Federform bestimmter Federpartien. Die identifizierten Küken werden separiert und in die jeweiligen Vermehrer- bzw. Produktionsbetriebe gebracht. Insbesondere werden männliche Küken der Legehenennenlinien nur in geringer Anzahl zur Zucht benötigt (Eltern-/Großelterntiere) bzw. sind aufgrund der Genetik nicht für die Mast geeignet und werden direkt nach dem Schlupf aussortiert und getötet (Endprodukt).

Das standardmäßige Töten männlicher Eintagsküken stößt gemäß dem deutschen Tierschutzgesetz zunehmend auf ethische und rechtliche Bedenken. Praxistaugliche Alternativen stehen bislang allerdings nicht zur Verfügung.

In herkömmlichen Vorrichtungen zur Geschlechtsbestimmung von bebrüteten Vogeleiern, wie z.B. in der WO 2011/088825 A1 oder der DE 10 2007 013 107 A1, wird bei der Bearbeitung einer Kalkschale eine Lochausbildung in der Kalkschale außerhalb des Bereiches der Luftkammer mit Öffnung der beiden Membranen vorgenommen, wobei eine starke Beeinflussung des Bruteies in Kauf genommen wird, und es treten oftmals Verletzungen auf, sodass nach der Geschlechtsbestimmung oftmals keine weitere Entwicklung des Embryos möglich ist, sodass der Schlupferfolg deutlich herabgesetzt wird.

Die US 2008/0289578 A1 offenbart ein Verfahren und eine Anordnung zur Detektion von Keimscheiben in befruchteten unbebrüteten Geflügeleiern.

Die US 2007/0137577 A1 offenbart ein Verfahren und eine Vorrichtung zum Injizieren und Entnehmen von Material, wobei eine Eischale geöffnet wird.

Die DE 699 05 493 T2 offenbart eine Methode zum Injizieren von einer Mehrzahl von Vogeleiern.

Die WO 2014/021715 A2 offenbart Methoden zur in-ovo-Bestimmung von Geschlecht, Lebensfähigkeit und/oder Entwicklungsstadium von Vogelembryonen.

Die US 2015/0136030 A1 offenbart ein ESTIS ("Embryonic Structure Targeting Injection System")-System zum Injizieren einer Substanz in eine bestimmte Stelle eines sich entwickelnden Vogelembryos oder in eine bestimmte Stelle eines Eis, das einen Vogelembryo enthält.

Der Erfindung liegt deshalb die Aufgabe zugrunde, ein Verfahren und eine Vorrichtung zur Einbringung einer Öffnung in die Kalkschale im Bereich des stumpfen Pols von bebrüteten Vogeleiern mit darin enthaltenem Embryo anzugeben, die derart geeignet ausgebildet sind, dass die Öffnung in der Kalkschale als freier Zugang zur Luftkammer, ohne schadhafte Beeinflussung der inneren Membran der Kalkschale, entsteht und damit eine Weiterentwicklung des Embryos gewährleistet werden kann. Ferner besteht die Aufgabe, ein Verfahren und eine Vorrichtung zur Geschlechtsbestimmung von Vogelembryos bereitzustellen, bei denen die Gefahr einer Schädigung des Embryos verringert bzw. vermieden wird.

Diese Aufgaben werden durch die Merkmale der unabhängigen Patentansprüche gelöst. Die abhängigen Patentansprüche beziehen sich auf weitere Aspekte der Erfindung.

Durch die vorliegende Erfindung wird zum einen eine möglichst schonende Öffnung der bebrüteten Vogeleier ermöglicht, ohne dabei die innere Membran zu beschädigen. Durch die Öffnung ist es möglich das Geschlecht der Embryos nicht-invasiv, z.B. mit Hilfe optischer Verfahren, beispielsweise spektroskopischer Verfahren, wie der Raman-Spektroskopie und/oder Fluoreszenzspektroskopie, zu bestimmen. Nach der Geschlechtsbestimmung können die Vogeleier erfindungsgemäß wieder verschlossen werden. Somit kann eine Weiterentwicklung des Embryos gewährleistet werden.

Als wesentlicher Vorteil bildet sich heraus, dass durch den Erhalt der inneren Membran das Eiinnere bzw. der sich entwickelnde Embryo weitgehend unbeeinflusst bleibt und im Vergleich zu anderen Öffnungsverfahren höhere Schlupfraten ermöglicht.

Gemäß eines Aspekts der vorliegenden Erfindung wird ein Verfahren zur Einbringung einer Öffnung in die Kalkschale eines bebrüteten Vogeleis mit darin enthaltenem Embryo, im Bereich des stumpfen Pols des bebrüteten Vogeleis bereitgestellt, wobei sich innerhalb des Bereiches des stumpfen Pols eine äußere Membran und eine innere Membran befinden, zwischen denen sich eine Luftkammer befindet. Das Verfahren weist die folgenden Schritte auf:
a) Lagern der bebrüteten Vogeleier mit dem spitzen Pol nach unten, wobei der Embryo angrenzend an die innere Membran anliegt;
b) Durchleuchten des bebrüteten Vogeleis und Detektieren des durch das bebrütete Vogelei hindurchgetretenen Lichtes zum Bestimmen der Position und der Geometrie der Luftkammer am stumpfen Pol des bebrüteten Vogeleies; und
c) Anschließendes Einbringen einer Öffnung in die Kalkschale am stumpfen Pol des bebrüteten Vogeleies oberhalb der aufgespannten inneren Membran zur Luftkammer, um einen Zugang zur Luftkammer zu erreichen.

Das Verfahren kann ferner einen Schritt zum Bestimmen der Positionen und Geometrie des bebrüteten Vogeleis aufweisen, wobei sich das bebrütete Vogelei vorzugsweise auf einer vorgegebenen Bruthorde befindet.

Der Schritt zum Bestimmen der Position und der Geometrie der Luftkammer kann einen Schritt zum Bestimmen einer zweidimensionale Projektion der Luftkammer mit einem zentralen Punkt m aus dem detektierten, durch das bebrütete Vogelei hindurchgetretenen Licht aufweisen, wobei die zweidimensionale Projektion der Luftkammer eine im Wesentlichen elliptische, gegebenenfalls kreisförmige, Form mit dem Schnittpunkt von sich schneidenden Ellipsenhauptachsen A, B als zentralen Punkt m aufweist.

Der zentrale Punkt m kann als Mittelpunkt für das Einbringen der Öffnung verwendet werden, wobei die Öffnung vorzugsweise kreisförmig ist und einen Radius R besitzt, der höchstens der Hälfte der kürzeren Ellipsenhauptachse entspricht.

Gemäß der vorliegenden Erfindung kann das Einbringen der Öffnung einen Schritt zum Einbringen einer Sollbruchstelle in die Kalkschale aufweisen, wobei das Einbringen der Öffnung einen Schritt zum Entfernen des durch die Sollbruchstelle definierten Bereichs der Kalkschale aufweisen kann.

Das Verfahren kann ferner nach dem Einbringen der Öffnung einen weiteren Schritt zum Durchleuchten des bebrüteten Vogeleis und einen Schritt zum Detektieren des durch das bebrütete Vogelei hindurchgetretenen Lichtes umfassen, wobei vorzugsweise Licht im Spektralbereich von 500nm bis 600nm verwendet wird, um die embryospezifischen Zielstrukturen zu erfassen.

Das Verfahren kann vor dem Detektieren des durch das bebrütete Vogelei hindurchgetretenen Lichtes einen Schritt zum Ermitteln des Abstandes a der inneren Membran vom Eizenit des stumpfen Pols aus und einen Schritt zum Fokussieren der inneren Membran unter Verwendung des Abstandes a aufweisen.

Das Verfahren kann ferner einen Schritt zum Bestimmen der Position des Embryos unter Verwendung des detektierten, durch das bebrütete Vogelei hindurchgetretenen, Lichtes aufweisen.

Ferner kann das Verfahren einen Schritt zum Bestimmen des Geschlechtes des Embryos aufweisen, wobei der Schritt zum Bestimmen des Geschlechtes des Embryos einen Schritt zum Messen optischer Daten, vorzugsweise unter Verwendung von Absorptionsspektroskopie, insbesondere Ramanspektroskopie oder Fluoreszenzspektroskopie, oder unter Verwendung von chemischer oder biologischer Daten, aufweisen kann.

Das Verfahren kann nach dem Einbringen der Öffnung ferner einen Schritt zum Verschließen der Öffnung, vorzugsweise mit einer semipermeablen Membran aus biokompatiblem Material, aufweisen.

Das Verfahren kann ferner einen Schritt zum Desinfizieren von mindestens dem stumpfen Pol des bebrüteten Vogeleies aufweisen.

Das Verfahren kann auch einen Schritt zum Zuführen der bebrüteten Vogeleier und einen Schritt zum Abführen der bebrüteten Vogeleier umfassen.

Gemäß eines weiteren Aspekts der vorliegenden Erfindung wird eine Vorrichtung zur Einbringung einer Öffnung in die Kalkschale eines bebrüteten Vogeleis mit darin enthaltenem Embryo, im Bereich des stumpfen Pols des bebrüteten Vogeleis bereitgestellt, wobei sich innerhalb des Bereiches des stumpfen Pols eine äußere Membran und eine innere Membran befinden, zwischen denen sich eine Luftkammer befindet, wobei die Vorrichtung vorzugsweise geeignet ist das oben beschriebene Verfahren durchzuführen. Die Vorrichtung weist dabei eine Haltevorrichtung auf, vorzugsweise eine Bruthorde, die konfiguriert ist, das bebrütete Vogelei mit dem spitzen Pol nach unten zu lagern, wobei der Embryo angrenzend an die innere Membran anliegt. Die Vorrichtung weist ferner eine erste Detektionseinrichtung auf, die konfiguriert ist, die Position und Geometrie der Luftkammer zu bestimmen, wobei die erste Detektionseinrichtung eine erste Durchleuchtungseinrichtung, die konfiguriert ist, Licht durch das bebrütete Vogelei zu senden, und einen ersten Detektor aufweist, der konfiguriert ist, das durch das bebrütete Vogelei transmittierte Licht aufzuzeichnen. Die Vorrichtung weist ferner eine Öffnungseinrichtung auf, die konfiguriert ist eine Öffnung in die Kalkschale am stumpfen Pol des bebrüteten Vogeleies oberhalb der aufgespannten inneren Membran zur Luftkammer einzubringen, um einen Zugang zu der Luftkammer zu schaffen.

Die erste Durchleuchtungseinrichtung kann unterhalb des bebrüteten Vogeleis angebracht sein und der erste Detektor kann oberhalb des bebrüteten Vogeleis und gegenüber der ersten Durchleuchtungseinrichtung angebracht sein.

Die erste Detektionseinrichtung kann ferner konfiguriert sein, die Position und Geometrie des mit dem spitzen Pol nach unten gelagerten bebrüteten Vogeleis zu erfassen, wobei die Vorrichtung vorzugsweise ferner eine Bruthorde umfasst, auf der das bebrütete Vogelei lagert.

Die erste Detektionseinrichtung kann ein Sensor oder ein Sensor-Array sein, und vorzugsweise einen Abstandssensor oder einen Triangulationssensor aufweisen.

Ferner kann die Vorrichtung eine Auswerte- und Steuereinheit aufweisen, die konfiguriert ist, aus dem detektierten Licht eine zweidimensionale Projektion der Luftkammer mit einem zentralen Punkt m zu bestimmen, wobei die zweidimensionale Projektion der Luftkammer eine im Wesentlichen elliptische, gegebenenfalls kreisförmige, Form mit dem Schnittpunkt von sich schneidenden Ellipsenhauptachsen A, B als zentralen Punkt m aufweist.

Die Auswerte- und Steuereinheit kann ferner konfiguriert sein, eine im Wesentlichen kreisförmige Öffnung zu bestimmen, wobei der Mittelpunkt der Öffnung dem zentralen Punkt m entspricht und vorzugsweise einen Radius R aufweist, der höchstens der Hälfte der kürzeren Ellipsenhauptachse entspricht.

Die Öffnungseinrichtung kann eine Bearbeitungseinrichtung aufweisen, die konfiguriert ist, eine Sollbruchstelle in Form der Öffnung einzubringen, wobei die Bearbeitungseinrichtung vorzugsweise eine laser-optische Einrichtung ist, die konfiguriert ist, mit einem Laserstrahl eine Perforation der Kalkschale durchzuführen.

Die Öffnungseinrichtung kann ferner eine Entfernungseinrichtung aufweisen, die konfiguriert ist, den durch die Sollbruchstelle definierten Bereich zu entfernen.

Die Vorrichtung kann auch eine zweite Durchleuchtungseinrichtung aufweisen, die unterhalb des bebrüteten Vogeleis angebracht ist und konfiguriert ist, Licht durch das bebrütete Vogelei zu senden, wobei vorzugsweise Licht im Spektralbereich von 500nm bis 600nm verwendet wird.

Die Vorrichtung kann ferner einen zweiten Detektor aufweisen, der oberhalb des bebrüteten Vogeleis und gegenüber der zweiten Durchleuchtungseinrichtung angebracht ist, wobei der zweite Detektor konfiguriert ist, das durch das bebrütete Vogelei transmittierte Licht aufzuzeichnen.

Die Auswerte- und Steuereinheit kann ferner konfiguriert sein, die Position des Embryos aus dem durch das bebrütete Vogelei transmittierten und von dem zweiten Detektor aufgezeichneten Licht zu bestimmen.

Die Vorrichtung kann ferner eine Geschlechtsbestimmungseinheit aufweisen, die konfiguriert ist das Geschlecht des Embryos zu bestimmen, wobei die Geschlechtsbestimmungseinheit eine optische Messeinheit, vorzugsweise eine Absorptionsspektroskopieeinheit, insbesondere eine Raman-Spektroskopieeinheit oder eine Fluoreszenzspektroskopieeinheit, oder eine chemische Messeinheit oder eine biologische Messeinheit sein kann.

Die Vorrichtung kann ferner eine Verschließungseinrichtung aufweisen, die konfiguriert ist, die Öffnung zu verschließen, vorzugsweise unter Verwendung einer semipermeablen Membran aus biokompatiblem Material.

Die Vorrichtung kann auch eine Desinfenktionseinrichtung aufweisen, die konfiguriert ist, mindestens den stumpfen Pol der bebrüteten Vogeleier zu desinfizieren.

Ferner kann die Vorrichtung eine Transporteinrichtung aufweisen, die konfiguriert ist, das bebrütete Vogelei in eine Transportrichtung zu transportieren, wobei die Transporteinrichtung ferner eine Zuführungseinrichtung aufweisen kann, die konfiguriert ist, das bebrütete Vogelei der Vorrichtung zuzuführen und wobei die Transporteinrichtung ferner eine Abführungseinrichtung aufweisen kann, die konfiguriert ist, das bebrütete Vogelei der Vorrichtung abzuführen.

Erfindungsgemäß wird ein Verfahren zur Einbringung einer Öffnung in die Kalkschale im Bereich des stumpfen Pols von mit dem spitzen Pol nach unten gelagerten bebrüteten Vogeleiern mit Embryo bereitgestellt. Innerhalb des Bereiches des stumpfen Pols befindet sich eine äußere Membran und eine innere Membran, zwischen denen sich eine Luftkammer befindet, wobei der Embryo angrenzend an die innere Membran anliegt. Die zu untersuchenden Bruteier können sich in einer vorgegebenen Einlage befinden. Ferner kann eine Vermessung des mit dem spitzen Pol nach unten gerichteten und gelagerten Bruteies durchgeführt werden, wobei die Platzierung und die Abmaße der Luftkammer innerhalb des Bereiches des stumpfen Pols und die Lage des Embryos unterhalb der den Embryo von der Luftkammer abschirmenden inneren Membran festgestellt werden können. Eine zweidimensionale Projektion des Bruteies mit einem Zentrum M und damit überlagert eine zweidimensionale Projektion der Luftkammer mit einem zentralen Punkt m kann durchgeführt werden, wobei die zweidimensionale Projektion der Luftkammer z.B. eine elliptische Form mit dem Schnittpunkt m von sich schneidenden Ellipsenhauptachsen A, B aufweist. Dem Schnittpunkt m der Ellipsenhauptachsen A, B kann der Mittelpunkt einer der beabsichtigten Öffnung zuzuordnenden Bruchstellenprojektion zugeordnet werden. Ein der zweidimensionalen Bruchstellenprojektion entsprechender Kalkschalendeckel kann von dem restlichen Körper der Kalkschale gelöst und abgetragen werden. Eine Öffnung in der Kalkschale und in der an der Kalkschale haftenden äußeren Membran und somit ein Zugang zur festgestellten Luftkammer kann somit erreicht werden.

Die Bruchstellenprojektion für die Sollbruchstelle kann eine Kreisprojektion darstellen, wobei der Radius R der Kreisprojektion kleiner als die halbe Ausdehnung A/2 der kleinen Ellipsenhauptachse A mit R < A/2 ist.

Vor Beginn der Vermessung der Bruteier werden die Bruteier in der Einlage vorzugsweise im Bereich des stumpfen Pols desinfiziert.

Vorzugsweise können danach einer oder mehrere der folgenden Schritte unter Einsatz einer Auswerte- und Steuereinheit durchgeführt werden: Ermittlung der Positionen und Geometrien der mit dem spitzen Pol nach unten gelagerten Bruteier, die sich auf einer vorgegebenen Bruthorde befinden, Ermittlung der Geometrie der Luftkammer am stumpfen Pol des Bruteies, Detektierung und Digitalisierung der Geometriedaten des Volumens der Luftkammer, Festlegung eines zweidimensionalen Umrisses in Form einer in eine Ebene projizierten Fläche in einem digitalisierten Kamerabild aus der Volumenprojektion der Luftkammer in Form einer Ellipse, Berechnung des Schnittpunktes m der Ellipsenhauptachsen A, B der Ellipse aus dem digitalisierten Kamerabild, Berechnung der Bruchstellenprojektion und der zugehörigen grabenartigen Sollbruchstelle in Bezug auf den Schnittpunkt m der Ellipse, sowie Überlagerung der Bruchstellenprojektion auf die Projektion der Luftkammer. Einbringen einer Öffnung in die Kalkschale in der zentrisch zum Ellipsenschnittpunkt m liegenden Bruchstellenprojektion über die Sollbruchstelle oberhalb der aufgespannten inneren Membran mittels einer Bearbeitungseinrichtung, und Abtragen des abgetrennten Teils der Kalkschale als Deckel gemäß der definierten Sollbruchstelle und Schaffung der Öffnung.

Eine Ermittlung des Abstandes a der inneren Membran vom Eizenit des stumpfen Pols aus kann durchgeführt werden, wobei der Abstand a zur Fokussierung der Farbkamera auf eine Zielstruktur im Bereich der inneren Membran genutzt wird.

Nach der Öffnung der Kalkschale kann eine erneute zweite Durchleuchtung des Bruteies und eine Aufnahme des festgelegten Bereiches der inneren Membran der Luftkammer mittels einer Farbkamera zur Festlegung der Zielstruktur für eine Aufnahme von Ei- und embryonalen Messdaten der Zielstruktur durchgeführt werden.

Die Aufnahme von Ei- und embryonalen Messdaten der Zielstruktur kann durch Einsatz der Absorptionsspektroskopie, wie z.B. der Ramanspektroskopie oder auch der Fluoreszenzspektroskopie durchgeführt werden.

Eine Einstellung einer angepassten Kontrastierung der unter der inneren Membran liegenden, embryospezifischen Zielstrukturen kann unter Einsatz einer Lichtquelle, vorzugsweise im Spektralbereich von 500nm bis 600nm, mit der zweiten Durchleuchtungseinrichtung durchgeführt werden.

Nach Aufnahme und Messung der ei- und embryospezifischen Merkmale kann eine Verschließung der Öffnung der Luftkammer mittels eines Verschlusselements in Form einer semipermeablen Membran durchgeführt werden.

Mindestens eine Einlage mit Bruteiern, in der die Bruteier gehaltert einsortiert werden, kann der Transporteinrichtung zugeordnet werden.

Als erfassbarer Bereich der Öffnung kann eine Markierung an einer Einlage für die Bruteier oder an der Transporteinrichtung ausgewählt werden, wenn die im Bereich der Zuführungseinrichtung installierte Positioniereinrichtung auf die Erfassung der Position der Einlage ausgerichtet wird und wenn die erste Positioniereinrichtung oberhalb der Einlage angeordnet wird.

Die Einbringung der Öffnung im Kalkschalenbereich stellt eine definierte Bearbeitung am Brutei dar, wobei die Ausgangspositionierung mittels eines Sensors oder eines Sensor-Arrays und die zur Öffnungseinbringung zugeordnete Endpositionierung des Bruteies, die mit der zugehörigen Einstellpositionierung der Bearbeitungseinrichtung übereinstimmt, mittels des Einsatzes von in der Auswerte- und Steuereinheit gespeicherten programmtechnischen Mitteln durchgeführt wird.

Mit den erhaltenen Daten eines Abstandssensors oder eines Triangulationssensors oder Streiflichtsensors, wobei die Daten jeweils dem zugeführten Brutei zugeordnet werden, kann eine genaue Positionierung der Bearbeitungseinrichtung an einer für die Öffnungseinbringung vorgegebenen, standortveränderlichen Position in Bezug auf den zu bearbeitenden Kalkschalenbereich des Bruteies eingestellt werden.

Die Einlage in Form einer Horde für Bruteier kann mittels der Transporteinrichtung unterhalb der ersten Detektionseinrichtung und der Bearbeitungseinrichtung zur Einbringung der Öffnung vorbeibewegt werden, wobei mittels der ersten Detektionseinrichtung von der Draufsicht des Bruteies ein 2D-Bildbereich oder ein 3D-Bildbereich erfasst wird, dessen Daten zur weiteren Verarbeitung in die Auswerte- und Steuereinheit über die elektrischen Verbindungsleitungen geleitet werden. In der Auswerte- und Steuereinheit können die Bilddaten des 2D-Bildbereiches gemeinsam mit den Eiabstandsdaten des Abstandssensors oder des Triangulationssensors mittels aktivierter programmtechnischer Mittel verarbeitet werden und die verarbeiteten erhaltenen Signale an die Bearbeitungseinrichtung zur Einbringung einer Sollbruchstelle weitergeleitet werden.

Die von außen durchgeführte Öffnung der Kalkschale des Bruteies im Bereich der Luftkammer kann auch mittels mechanischer, chemischer oder Wasserstrahl-Werkzeuge durchgeführt werden.

Zur erfindungsgemäßen Vorrichtung können folgende Baugruppen gehören: eine Zuführungseinrichtung, eine Transporteinrichtung zum Transport der Einlage, eine Positioniereinrichtung zur Festlegung der Standorte und der Lage der Bruteier in der Einlage, eine erste Detektoreinrichtung zur Erfassung der Eiabmaße, eine zweite Detektionseinrichtung mit einer ersten Durchleuchtungseinrichtung und einer Farbkamera zur Erfassung der Abmaße der Luftkammer, und eine Bearbeitungseinrichtung, die eine in einer Auswerte- und Steuereinheit berechnete Sollbruchstelle in der Kalkschale längs der grabenartigen Sollbruchstelle abträgt und als einen abzuhebenden Deckel realisiert. Ferner können zur Vorrichtung folgende Baugruppen gehören: eine Einrichtung zum Abheben und Entfernen des Deckels und zur Schaffung einer Öffnung in der Luftkammer, eine Farbkamera mit einer zweiten Durchleuchtungseinrichtung zur Erfassung und Fokussierung der zu untersuchenden Zielstruktur im Bereich der inneren Membran innerhalb der Luftkammer, eine Einheit zur Aufnahme von Ei- und embryonalen Messdaten der Zielstruktur mit einer Messsonde im Strahlengang, der auf die Zielstruktur gerichtet wird, eine Verschließungseinrichtung, die die offene Luftkammer mit einem Verschlusselement verschließt, eine Abführungseinrichtung und eine Auswerte- und Steuereinheit, die mit den genannten Baugruppen in signaltechnischer Verbindung, z.B. über Verbindungsleitungen steht und mit einem Algorithmus die Durchführung der Öffnung zur Luftkammer leitet.

Die Einheit zur Aufnahme von Ei- und embryonalen Messdaten der Zielstruktur kann eine Absorptions-Spektren-Aufnahmeeinheit, wie z.B. eine Raman-Spektren-Aufnahmeeinheit oder eine Fluoreszenz-Spektren-Aufnahmeeinheit darstellen.

Die Vorrichtung kann ferner eine Transporteinrichtung, mit der eine Zuführung und eine Abführung mindestens eines Bruteies innerhalb der Vorrichtung erfolgt, eine erste Detektionseinrichtung zur Erfassung eines auf die Bruteier bezogenen Bereiches und zur Umwandlung der Daten des Bereiches in elektrische Daten, eine zweite Detektionseinrichtung zur Erfassung der Abmaße der Luftkammer mit der ersten Durchleuchtungseinrichtung, eine Bearbeitungseinrichtung zur Einbringung einer Sollbruchstelle zur Schaffung einer Öffnung in der Kalkschale, die zur Bearbeitung Bearbeitungssignale aus der Auswerte- und Steuereinheit erhält, eine Einrichtung zur Abhebung des Deckels längs der Sollbruchstelle, wodurch die Öffnung in der Luftkammer entsteht, eine Farbkamera zur Fokussierung auf eine Zielstruktur der inneren Membran mittels der zweiten Durchleuchtungseinrichtung, eine Einheit zur Aufnahme von ei- und embryonalen Messdaten der Zielstruktur, eine Verschließungseinrichtung, und eine Auswerte- und Steuereinheit aufweisen.

Die in die Einheit zur Aufnahme von ei- und embryonalen Messdaten der Zielstruktur aufgenommenen Messdaten können beispielsweise optische Messdaten, vorzugsweise Messdaten aus der eingesetzten Absorptionsspektroskopie, wie z.B. der Ramanspektroskopie oder der Fluoreszenzspektroskopie, oder chemische oder biologische Messdaten darstellen.

Zumindest vor der Bearbeitungseinrichtung kann eine Einrichtung zur Desinfektion zumindest des Bereiches des stumpfen Pols angeordnet sein.

Das eingesetzte Verschlusselement kann aus biokompatiblem Material bestehen.

Als erste Detektionseinrichtung kann ein Sensor oder ein Sensor-Array eingesetzt sein, dem wahlweise ein Abstandssensor oder ein Triangulationssensor zugeordnet ist.

Der Transporteinrichtung kann mindestens eine Einlage/Horde/Schlupfhorde mit Bruteiern zugeordnet sein, in der die Bruteier gehaltert einsortiert sind.

Den standardisierten oder vorab vermessenen Einlagen mit konstanten Abständen A_{B}, A_{E}, A_{S} der Eiaufnahme-Ausbuchtungen zur Bestimmung der Positionsbereiche der einsortierten Bruteier kann eine Positioniereinrichtung zugeordnet sein, die nur auf die erforderliche Erfassung der Einlage ausgerichtet ist, sodass die Positionsbereiche der Bruteier aus den konstanten Abständen A_{B}, A_{E}, A_{S} ermittelbar sind.

Die Positioniereinrichtung kann zur Erfassung eines auf die Bruteier bezogenen Bereiches aus der Einlage oder eines Positionsbereiches mindestens eines Bruteies in der Einlage dienen, wobei die Positioniereinrichtung eine vorgegebene Markierung der Einlage erfasst, und wobei aus den festgelegten Abständen A_{B}, A_{E}, A_{S} der Eiaufnahme-Ausbuchtungen der Einlage zueinander und zur Markierung der Einlage der Positionsbereich des jeweiligen Bruteies in der Auswerte- und Steuereinheit ermittelt wird.

Die Vorrichtung kann des Weiteren mindestens einen als erste Detektionseinrichtung ausgebildeten Sensor oder ein Sensor-Array zur Erfassung eines 2D-Bildbereich oder eines 3D-Bildbereiches in Draufsicht auf den Kalkschalenbereich mindestens eines zugeführten Bruteies und wahlweise mindestens einen Abstandssensor oder einen Triangulationssensor aufweisen, wobei der Sensor die Positionsdaten des zugeführten Bruteies erfasst. Die Vorrichtung kann ferner eine Auswerte- und Steuereinheit umfassen, die die Eipositionsdaten und die Positionsdaten, vorzugsweise die Daten der Sollbruchstelle, der Bearbeitungseinrichtung zur Einbringung einer Sollbruchstelle aufnimmt und verarbeitet, wobei die Bearbeitungseinrichtung zur Einbringung der Sollbruchstelle in der Kalkschale die zur Bearbeitung erforderlichen ausführenden Bearbeitungssignale aus der Auswerte- und Steuereinheit erhält.

Die Bearbeitungseinrichtung zur Einbringung der Sollbruchstelle kann eine laser-optische Einrichtung sein, die mit ihrem Laserstrahl eine Perforation der Kalkschale durchführt und eine grabenartige Sollbruchstelle ausbildet.

Die Transporteinrichtung kann als Zuführungseinrichtung und als Abführungseinrichtung in durchgängiger Form ausgebildet sein.

Der Sensor oder das Sensor-Array und die Bearbeitungseinrichtung können oberhalb der Transporteinrichtung sowie oberhalb der in der transportierten Einlage befindlichen Bruteier angeordnet sein.

Im Prinzip kann die gesamte Anordnung der angegebenen Baugruppen eine automatische Bearbeitungslinie darstellen.

Gemäß dem erfindungsgemäßen Verfahren können vorzugsweise folgende Schritte durchgeführt werden: Durchlicht-Bestrahlung und Projektion für die Darstellung der Position der Luftkammer in dem bebrüteten Vogelei mit Embryo, Einbringung einer Öffnung in die Kalkschale, wobei die Öffnung zentral auf die Fläche der inneren Membran projiziert wird, und Verschließung der geöffneten Luftkammer mittels mindestens einer semipermeablen Membran zur Gewährleistung des Gasaustausches der Luftkammer mit der Außenluft nach der Beendigung der Durchführung der Messaufnahme, insbesondere des spektroskopischen Verfahrens, und Beibehaltung der Keimfreiheit.

Da sich berührungsfrei arbeitende laser-optische Verfahren ideal in die Prozesse der Geflügelindustrie eingliedern lassen, wird vorzugsweise eine auf einem CO₂-Laser bzw. einem Nd:YAG-Laser oder Er:YAG-Laser basierende Vorrichtung zur Bearbeitung der Kalkschale von Bruteiern eingesetzt. Andere Laserverfahren können auch eingesetzt werden.

Die Erfindung wird anhand eines Ausführungsbeispiels mittels der Zeichnungen näher erläutert.

Es zeigen:
- Fig. 1: eine schematische Darstellung einer erfindungsgemäßen Vorrichtung zur Einbringung einer Öffnung in die Kalkschale von bebrüteten Vogeleiern mit darin enthaltenem Embryo im Bereich des stumpfen Pols mit mehreren Baugruppen oberhalb von einer mit Bruteiern gefüllten Einlage, insbesondere zur Erzeugung einer Öffnung in Richtung zur Luftkammer im Bereich des stumpfen Pols eines bebrüteten Vogeleis mit Embryoausbildung, und zur Bestimmung des Geschlechts des Embryos,
- Fig. 2: eine schematische Schnittdarstellungen von Vogeleiern mit Vogelembryo mit möglicher Ausbildungen der inneren Membran, wobei die auf Bruthorden gelagerten Eier von unten, vom spitzen Ende ausgehend, von unterhalb der Bruthorde angeordneten Lichtquellen, vorzugsweise mittels weißen Lichts, durchleuchtet werden,
- Fig. 3: eine schematische Darstellung einer Projektion des Eies und der Projektion der zugehörigen Luftkammer,
- Fig. 4: eine schematische Darstellung gemäß Fig. 3, mit einer in den Mittelpunkt der Luftkammerprojektion festgelegten Kreisöffnung als Sollbruchstelle zur Ausbildung der Öffnung der Kalkschale, um einen Zugang zur Luftkammer zu schaffen,
- Fig. 5: eine schematische Darstellung eines vermessenen Eies mittels eines optischen Abstandsmessers zur Messung des Abstandes a der inneren Membran vom Eizenit des stumpfen Pols zur Fokussierung auf die Zielstruktur mit einer Aufnahme und einer Detektion charakteristischer Merkmale der Zielstruktur und
- Fig. 6: ein Raman-Spektrum, das erfindungsgemäß an der intakten inneren Membran der Luftkammer im Bereich des stumpfen Pols gemessen wird.

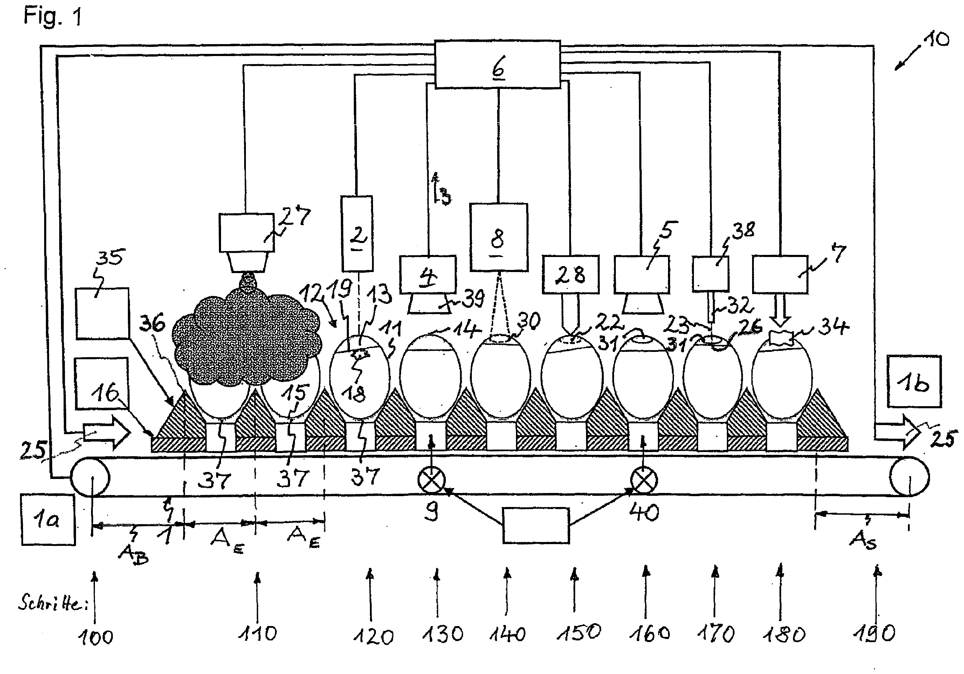

Im Folgenden wird die Funktionsweise der erfindungsgemäßen Vorrichtung und des erfindungsgemäßen Verfahrens anhand der Fig. 1 bis Fig. 6 näher erläutert.

In Fig. 1 ist eine Vorrichtung 10 zur Einbringung einer Öffnung 31 in die Kalkschale 11 im Bereich des stumpfen Pols 14 von bebrüteten Vogeleiern 12 mit darin enthaltenem Embryo 18 dargestellt. Innerhalb des Bereiches des stumpfen Pols 14 befindet sich eine äußere Membran 20 und eine innere Membran 19, zwischen denen sich eine Luftkammer 13 befindet. Der Embryo 18 liegt angrenzend an die innere Membran 19 beim mit dem spitzen Pol 15 nach unten gelagerten Brutei 12 an. Die Bruteier 12 werden in einer Einlage 16 gehaltert einsortiert. Die beispielhafte Ausführungsform gemäß Fig. 1 umfasst eine Transporteinrichtung 1, der die Einlage 16 in Form einer Horde zugeordnet ist, in der die Bruteier 12 mit dem stumpfen Pol 14 nach oben einsortiert sind, wobei die Einlage 16 derart ausgebildet ist, dass die Bruteier 12 in gleichen Abständen A_{E} an den Positionierbereichen 37 gelagert sind, und eine Positioniereinrichtung 35, die einen Marker 36 oder eine Markierung oder eine Kante an der Einlage 16 erfasst, aus denen die vorgegebenen Positionsbereiche 37 der Bruteier 12 über vorgegebene Abstände A innerhalb der Einlage 16 ermittelt werden. Die Vorrichtung umfasst ferner mindestens eine Detektionseinrichtung 2, die die Abmaße (Oberflächengeometrie) des Bruteies 12 erfasst, eine Detektionseinrichtung 4 zur Erfassung der Abmaße der Luftkammer 13 mit einer Durchleuchtungseinrichtung 9 und einer Farbkamera 39, eine Bearbeitungseinrichtung 8 zur Erzeugung einer Sollbruchstelle 30 in der Kalkschale 11, wobei nach der Bearbeitung der Kalkschale 11 die Kalkschale 11 grabenartige Sollbruchstellen 30 aufweist, und eine Einrichtung 28 zum Abheben und Entfernen (Entfernungseinrichtung) des von der Sollbruchstelle 30 definierten Deckels 22 und Schaffung einer Öffnung 31 in der Luftkammer 13. Desweiteren umfasst die Vorrichtung gemäß Fig. 1 eine Farbkamera 5 mit einer zweiten Durchleuchtungseinrichtung 40 zur Erfassung und Fokussierung der zu untersuchenden Struktur 26 im Bereich der inneren Membran 19 innerhalb der Luftkammer 13, eine Raman-Spektren-Aufnahmeeinheit 38 mit einer Messsonde 32 im Strahlengang 23, die auf die Struktur 26 gerichtet wird, eine Verschließungseinrichtung 7 mit Verschlusselementen 34 und eine Auswerte- und Steuereinheit 6, die mit allen Baugruppen jeweils über eine Verbindungsleitung verbunden ist.

Im Allgemeinen sind, wie in Fig. 1 gezeigt ist, die Einlagen/Bruthorde 16 z.B. palettenartig derart ausgebildet, dass die Abstände A_{E} zwischen den Bruteiern 12 selbst und die Abstände A_{B} (Beginn der Einlage) und die Abstände A_{S} (Schluss der Einlage) zwischen den Bruteiern 12 und z.B. einem Rand der Einlage 16 standardisiert sind und die standardisierten Werte bereits in der Auswerte- und Steuereinheit 6 gespeichert vorhanden sind oder sein können, sodass sie signaltechnisch problemlos zur Einstellung der jeweiligen Position der Öffnungseinrichtung 8 zur Einbringung der Öffnung 31 für die Berechnung/Ermittlung der zugeordneten Kalkschalenbereiche der Bruteier 12 verarbeitet werden können.

Das heißt, dass vorzugsweise für standardisierte oder vorab vermessene Bruthorden 16 mit konstanten Abständen A_{B}, A_{E}, A_{S} der Eiaufnahme-Ausbuchtungen zur Bestimmung der Positionsbereiche 37 der einsortierten Bruteier 12 eine Positioniereinrichtung 35 eingesetzt sein kann, die nur auf die erforderliche Erfassung der Bruthorde 16 ausgerichtet ist, sodass die Positionsbereiche 37 der Bruteier 12 aus den konstanten Abständen A_{B}, A_{E}, A_{S} ermittelbar sind.

Zusätzlich zur, oder einschließlich, der Detektionseinrichtung 2 in Form eines Sensors oder eines Sensor-Arrays kann wahlweise ein Abstandssensor oder ein Triangulationssensor eingesetzt sein. Die über die Detektionseinrichtung 2 hinaus angeordneten Sensoren dienen im Wesentlichen zur Absicherung der Genauigkeit bei einem kontinuierlichen Prozessverlauf.

Der von der Detektionseinrichtung 4 mit einer Kamera 39 in Verbindung mit einer ersten Einrichtung 9 zur Durchleuchtung des Eies 12 mit weißem Licht erfasste Bereich der Luftkammer 13 kann ein 2D-Bildbereich 3 in Form einer zweidimensionalen Projektion oder ein 3D-Bildbereich, der zu einer zweidimensionalen Projektion modifiziert wird, sein.

Der 2D-Bildbereich 3 liegt der Steuer- und Auswerteeinheit 6 zur Verarbeitung zugrunde, wobei in der Steuer- und Auswerteeinheit 6 die Projektion der Luftkammer 13 der Projektion des Eies 12 zugeordnet wird, und der Projektion der Luftkammer 13 eine Kreisprojektion 21 überlagert wird, wobei die Kreisprojektion 21 die Abmaße der Eingangsfläche der Öffnung 31 bestimmt.

Erfindungsgemäß enthalt die Vorrichtung 10 z.B. eine Zuführungseinrichtung 1a, eine Transporteinrichtung 1 zum Transport der Einlage 16, eine Positioniereinrichtung 35 zur Festlegung der Standorte der Bruteier 12 in der Einlage 16 und eine Detektionseinrichtung 2 zur Erfassung der Eiabmaße. Die Vorrichtung 10 kann ferner eine Detektionseinrichtung 4 mit einer ersten Durchleuchtungseinrichtung 9 und einer Farbkamera 39 zur Erfassung der Abmaße der Luftkammer 13, eine Bearbeitungseinrichtung 8, die eine in einer Auswerte- und Steuereinheit 6 berechnete Sollbruchstelle 30 in der Kalkschale 11 längs der Sollbruchstelle 30 abträgt und als einen abzuhebenden Deckel 22 realisiert, eine Einrichtung 28 zum Abheben und Entfernen des Deckels 22 und Schaffung einer Öffnung 31 in der Luftkammer 13 und eine Farbkamera 5 mit einer zweiten Durchleuchtung 40 zur Erfassung und Fokussierung der zu untersuchenden Struktur 26 im Bereich der inneren Membran 19 innerhalb der Luftkammer 13 aufweisen. Desweiteren kann die Vorrichtung 10 eine Raman-Spektren-Aufnahmeeinheit 38 mit einer Messsonde 32 im Strahlengang 23, die auf die Zielstruktur 26 gerichtet wird, eine Verschließungseinrichtung 7, die die offene Luftkammer 13 mit einem Verschlusselement 34 verschließt, eine Abführungseinrichtung 1b und eine Auswerte- und Steuereinheit 6 aufweisen, die mit allen genannten Baugruppen in signaltechnischer Verbindung über Verbindungsleitungen steht und mit einem Algorithmus die Durchführung der Öffnung 31 zur Luftkammer 13 leitet.

Zumindest vor der Bearbeitungseinrichtung 8 kann eine Einrichtung 27 zur Desinfektion (Desinfektionseinrichtung) zumindest des Bereiches des stumpfen Pols 14 angeordnet sein.

Der Bearbeitungseinrichtung 8 ist eine Verschließungseinrichtung 7 mit Verschlussmaterial zum Verschließen der geöffneten Öffnung 31 der Luftkammer 13 nachgeordnet.

Das Verschlusselement 34 kann aus biokompatiblem Material bestehen.

Als erste Detektionseinrichtung 2 kann ein Sensor oder ein Sensor-Array eingesetzt sein, dem wahlweise ein Abstandssensor oder ein Triangulationssensor zugeordnet ist.

Der Transporteinrichtung 1, 1a, 1b ist mindestens eine Einlage/Horde/Schlupfhorde 16 mit Bruteiern 12 zugeordnet, in der die Bruteier 12 gehaltert einsortiert sind.

Die Positioniereinrichtung 35 erfasst gemäß Fig. 1 eine vorgegebene Markierung 36 der Einlage 16 und ermittelt aus den festgelegten Abständen A_{B}, A_{E}, A_{S} der Eiaufnahme-Ausbuchtungen der Einlage 16 die Positionen der Eier 12 zueinander und ermittelt zur Markierung 36 der Einlage 16 den jeweiligen Positionsbereich 37 des jeweiligen Bruteies 12 in der Auswerte- und Steuereinheit 6.

Die Vorrichtung 10 kann mindestens einen als Detektionseinrichtung 18a ausgebildeten Sensor 2 oder Sensor-Array zur Erfassung eines 2D-Bildbereiches 3 oder eines 3D-Bildbereiches in Draufsicht auf den Kalkschalenbereich mindestens eines zugeführten Bruteies 12 und wahlweise mindestens einen Abstandssensor oder einen Triangulationssensor aufweisen, wobei der Sensor 2 die Positionsdaten des zugeführten Bruteies 12 erfasst und eine Auswerte- und Steuereinheit 6, die die Eipositionsdaten und die Positionsdaten der Bearbeitungseinrichtung 8 zur Einbringung einer Öffnung 31 aufnimmt und verarbeitet, wobei die Bearbeitungseinrichtung 8 zur Einbringung der Öffnung 31 in der Kalkschale 11 die zur Bearbeitung erforderlichen und auszuführenden Bearbeitungssignale aus der Auswerte- und Steuereinheit 6 erhält.

Die Transporteinrichtung 1 kann als Zuführungseinrichtung 1a und als Abführungseinrichtung 1b in durchgängiger Form ausgebildet sein.

Der Sensor 2 oder das Sensor-Array und die Bearbeitungseinrichtung 8 können z.B. oberhalb der Transporteinrichtung 1, 1a, 1b sowie oberhalb der in der transportierten Einlage 16 befindlichen Bruteier 12 angeordnet sein.

Die Bearbeitungseinrichtung 8 zur Erzeugung der Sollbruchstelle 30 kann in Fig. 1 eine laser-optische Einrichtung sein, die in der Kalkschale 11 eine Sollbruchstelle 30 mittels einer Perforation der Kalkschale 11 und finalem Durchbruch durchführt. Die laser-optische Einrichtung 8 kann auch als nachführbare, bewegbare Einrichtung ausgebildet sein.

Danach ist eine Einrichtung 28 zur Abhebung des freigeschnittenen Deckels 22 angeordnet.

Darauf folgt eine Farbkamera 5 einer zweiten Einrichtung 40 zur zweiten Durchleuchtung des Bruteis 12 zur Feststellung des Messortes auf der inneren Membran 19.

Der nachfolgend angeordneten Raman-Spektren-Aufnahmeeinheit 38 ist eine Streustrahlung aufnehmende Raman-Messsonde 32 zugeordnet, die die Ramanstreustrahlung über den Strahlengang 23 aufnimmt.

Mit der nachfolgend angeordneten Verschließungseinrichtung 7 wird ein Verschlusselement 34 auf die Öffnung 31 zum Verschließen der Öffnung 31 aufgebracht.

Die Zuführungseinrichtung 1a und die Abführungseinrichtung 1b sind in Fig. 1 Teil einer durchgängigen Transporteinrichtung 1, die eine vorgegebene Laufrichtung 25 aufweist.

In der Auswerte- und Steuereinheit 6 sind zur Positionierung und Auslösung der Öffnungsbearbeitung programmtechnische Mittel eingespeichert, die den Prozessablauf nach einem festgelegten Algorithmus abarbeiten, wobei die Auswerte- und Steuereinheit 6 mit allen Baugruppen der Einrichtungen in elektrischer Verbindung steht.

Der Sensor oder das Sensor-Array als Detektionseinrichtung 2 für die Eiabmaße sendet den 2D-Bildbereich 3 der erfassten Draufsicht des Bruteies 12 der Auswerte- und Steuereinheit 6 über vorgesehene elektrische Verbindungsleitungen zu. Der Sensor 2 oder das Sensor-Array und die laser-optische Öffnungseinrichtung 8 sind oberhalb der Transporteinrichtung 1, sowie oberhalb der in der Bruthorde/Einlage 16 befindlichen Bruteier 12, angeordnet.

Mit Hilfe der Detektionseinrichtung 4 für die Luftkammer in Form eines Abstandssensors oder eines Triangulationssensors wird eine genaue Positionierung der laser-optischen Bearbeitungseinrichtung 8 über dem Brutei 12 eingestellt. Die laser-optische Öffnungseinrichtung 8 erhält dazu Positionierungs- und Bearbeitungssignale von der Steuereinheit der Auswerteeinheit 6, die z.B. ein PC sein kann.

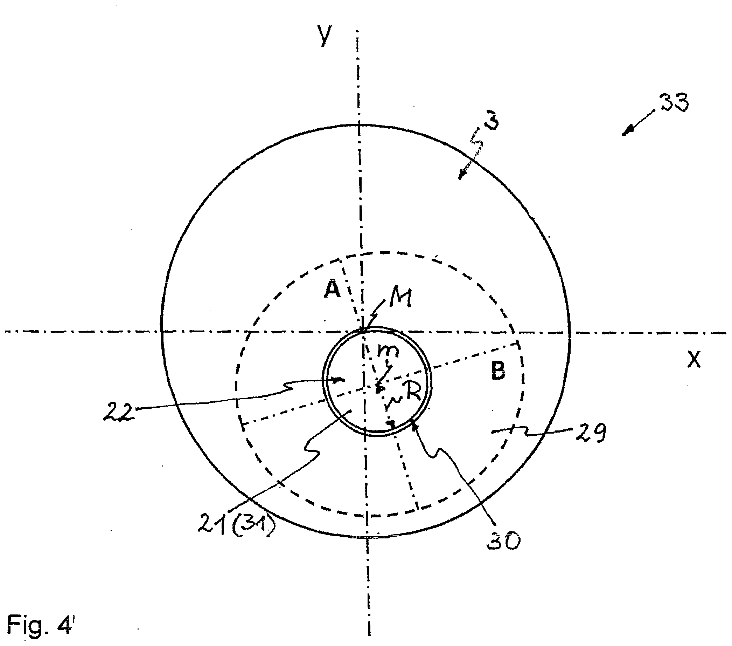

Im Allgemeinen befindet sich die Luftkammer 13 im Bereich des stumpfen Pols 14. Durch das Einbringen eines der Sollbruchstelle 30 entsprechenden Musters, wie in Fig. 4 gezeigt ist, kann ein Kalkschalendeckel 22 gezielt abgehoben werden.

Die von außen eingebrachte Öffnung kann gemäß Fig. 4 eine weitgehend den innereilichen Grenzen 17 der Luftkammer 13 angepasste Führungsbahn 30 in Form der Sollbruchstelle aufweisen. Diese kann beispielsweise mittels einer Durchlichttechnik ermittelt werden.

Damit können an unterschiedlichen Stellen positionierte Sollbruchstellen 30 auf/in die Kalkschale 11 im Bereich der Luftkammer 13 eingebracht sein.

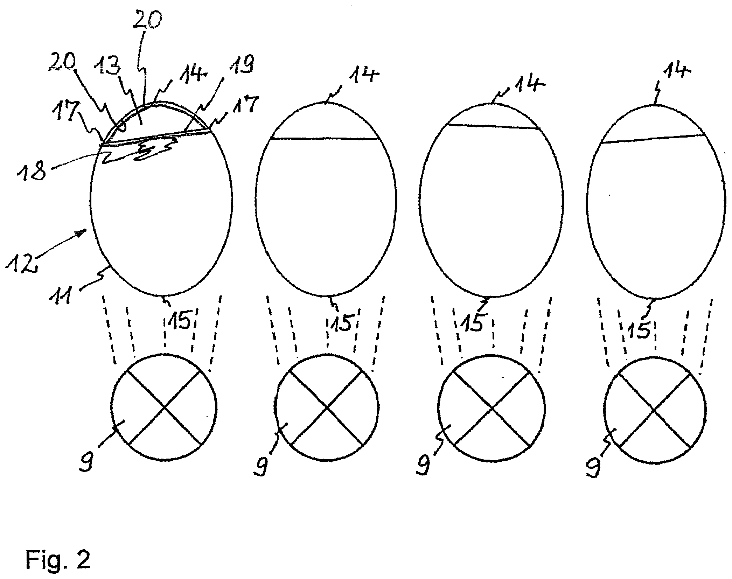

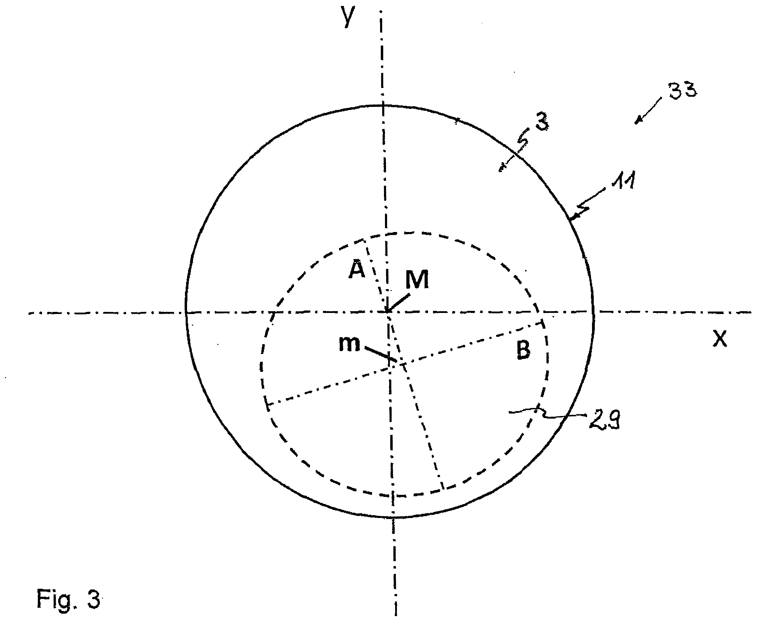

Das erfindungsgemäße Verfahren zur Einbringung einer Öffnung 31 in die Kalkschale 11 im Bereich des stumpfen Pols 14 von bebrüteten Vogeleiern 12 mit Embryo 18 wird nachfolgend anhand eines Ausführungsbeispiels beschrieben. Gemäß des Verfahrens der vorliegenden Erfindung wird eine Vermessung des mit dem spitzen Pol 15 nach unten gerichteten und gelagerten Bruteies 12 durchgeführt. Die Platzierung und die Abmaße der Luftkammer 13 innerhalb des Bereiches des stumpfen Pols 14 und die Lage des Embryos 18 unterhalb der den Embryo 18 von der Luftkammer 13 abschirmenden inneren Membran 19 wird festgestellt. Wie in den Figuren 2 und 3 gezeigt, wird eine zweidimensionale Projektion 3 des Bruteies 12 mit einem Zentrum M und damit überlagert eine zweidimensionale Projektion 29 der Luftkammer 13 mit einem zentralen Punkt m durchgeführt. Die zweidimensionale Projektion 29 der Luftkammer 13 hat vorzugsweise eine elliptische Form mit einem Schnittpunkt als zentralen Punkt m von sich schneidenden Ellipsenhauptachsen A, B. Dem Schnittpunkt m der Ellipsenhauptachsen A, B wird der Mittelpunkt einer die Sollbruchstelle 30 darstellenden Kreisprojektion 21 zugeordnet. Der Radius R der Kreisprojektion 21 ist vorzugsweise kleiner als die halbe Ausdehnung A/2 der kleinen Ellipsenhauptachse A mit R < A/2. Ein der zweidimensionalen Kreisprojektion 21 entsprechender Kalkschalendeckel 22 wird von dem restlichen Körper der Kalkschale 11 gelöst und abgetragen. Somit wird eine Öffnung 31 in der Kalkschale 11 und somit zur Luftkammer 13 erreicht.

Vor Beginn der Vermessung der Bruteier 12 werden die Bruteier 12 vorzugsweise zumindest im Bereich des stumpfen Pols 14 desinfiziert.

Das erfindungsgemäße Verfahren umfasst dabei vorzugsweise die folgenden Schritte: Ermittlung der Positionen und Geometrien der mit dem spitzen Pol 15 nach unten gelagerten und bereits desinfizierten Bruteier 12, die sich auf einer vorgegebenen Bruthorde 16 befinden, Ermittlung der Geometrie der Bruteier 12, Ermittlung der Geometrie der Luftkammer 13 am stumpfen Pol 14 des Bruteies 12 und Detektierung und Digitalisierung der Geometriedaten des Volumens der Luftkammer 13. Ferner kann das Verfahren die folgenden Schritte umfassen: Festlegung eines zweidimensionalen Umrisses 29 in Form einer in eine Ebene projizierten Fläche aus der Volumenprojektion der Luftkammer 13 in Form einer Ellipse, Berechnung des Schnittpunktes m der Ellipsenhauptachsen A, B der Ellipse 29 aus einem digitalisierten Kamerabild 33, Berechnung der Kreisprojektion 21 und der zugehörigen Sollbruchstelle 30 in Bezug auf den Schnittpunkt m der Ellipse, Einbringen einer Öffnung 31 in die Kalkschale 11 in der zentrisch zur Ellipse 29 liegenden Kreisprojektion 21 über die Sollbruchstelle 30 oberhalb der aufgespannten inneren Membran 19 mittels der Bearbeitungseinrichtung 8 und Abtragung des abgetrennten Teils der Kalkschale 11 als Deckel 22 in der definierten Kreisprojektion 21.

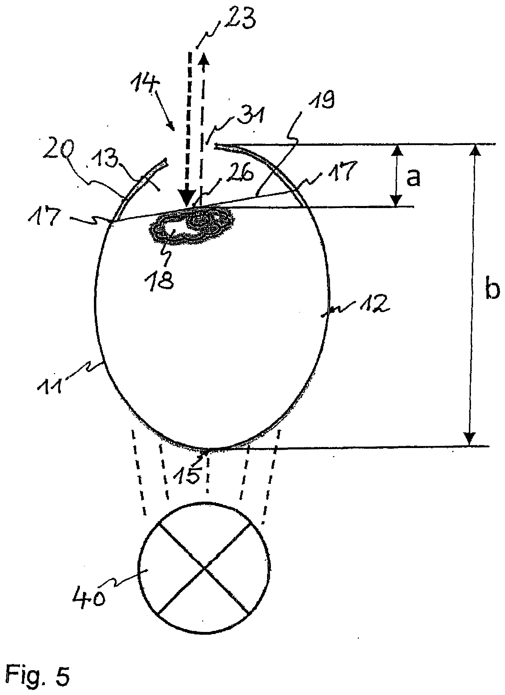

In Fig. 5 wird eine Ermittlung des Abstandes a der inneren Membran 19 vom Eizenit des stumpfen Pols 14 aus durchgeführt, wobei der erfasste Abstand a zur Fokussierung auf die Zielstruktur 26 im Bereich der inneren Membran 19 dient.

Nach der Öffnung der Kalkschale 11 wird eine erneute zweite Durchleuchtung des Bruteies 12 und eine Aufnahme des festgelegten Bereiches der inneren Membran 19 der Luftkammer 13 mittels einer Farbkamera 5 zur Festlegung der Struktur 26 für die Geschlechtsbestimmung, z.B. mittels Ramanspektroskopie durchgeführt.

Die Einstellung einer angepassten Kontrastierung der unter der inneren Membran 19 liegenden, embryospezifischen Strukturen 26 wird unter Einsatz einer Lichtquelle 40, vorzugsweise im Spektralbereich von 500nm bis 600nm, und der Farbkamera 5 durchgeführt.

Mit den erhaltenen Daten eines Abstandssensors oder eines Triangulationssensors oder Streiflichtsensors, wobei die Daten jeweils dem zugeführten Brutei 12 zugeordnet werden, wird eine genaue Positionierung der Bearbeitungseinrichtung 8 an einer für die Öffnungs-Einbringung vorgegebenen Position in Bezug auf den zu bearbeitenden Kalkschalenbereich 21 des Bruteies 12 eingestellt.

Die Einlage 16 in Form einer Horde der Bruteier 12 auf der Transporteinrichtung 1 wird unterhalb der Detektionseinrichtung 2 und der Bearbeitungseinrichtung 8 zur Einbringung der Öffnung 31 vorbeibewegt, wobei mittels der Detektionseinrichtung 2 von der Draufsicht des Bruteies 12 ein 2D-Bildbereich 3 oder ein 3D-Bildbereich, der in einen 2D-Bildbereich umgewandelt wird, erfasst wird, dessen Daten zur weiteren Verarbeitung in die Auswerte- und Steuereinheit 6 über elektrische Verbindungsleitungen geleitet werden, wobei in der Auswerte- und Steuereinheit 6 die Bilddaten des 2D-Bildbereiches gemeinsam mit den Eiabstandsdaten des Abstandssensors oder des Triangulationssensors mittels aktivierter programmtechnischer Mittel verarbeitet werden und die verarbeiteten erhaltenen Signale an die Bearbeitungseinrichtung 8 zur Einbringung der Öffnung 31 weitergeleitet werden.

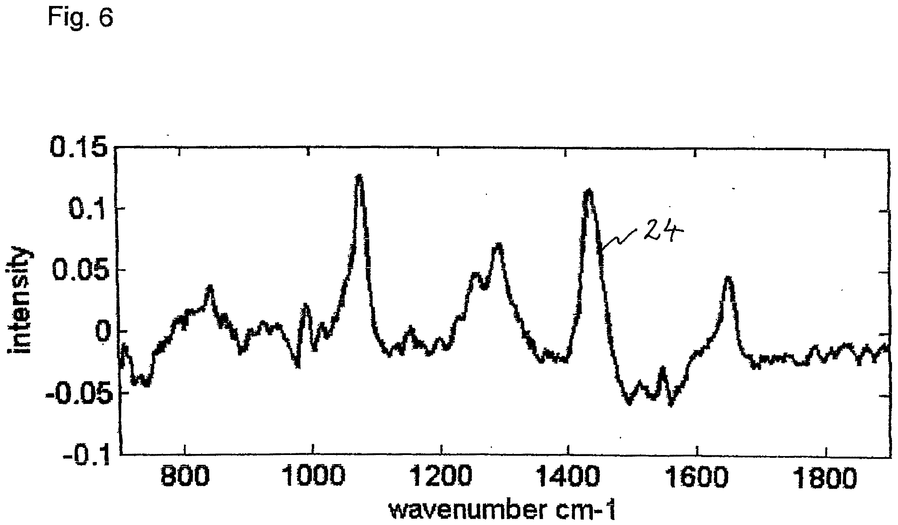

Das Verfahren zur Einbringung einer Öffnung 31 in die Kalkschale 11 im Bereich des stumpfen Pols 14 von bebrüteten Vogeleiern 12 mit darin enthaltenem Embryo 18 umfasst gemäß Fig. 1 folgende Schritte: Zuführen der Bruteier 12 mittels einer Zuführungseinrichtung 1a (Schritt 100), Desinfizieren zumindest des stumpfen Pols 14 (Schritt 110), Erfassen zumindest eines Positionsbereiches der zugeführten Bruteier 12 und/oder einer Bruteier 12 enthaltenen Einlage 16 mittels einer Positioniereinrichtung 35, die zumindest im Zuführungsbereich der Bruteier 12 enthaltenen Einlage 16 stationiert ist, und Umwandlung des jeweils erfassten Positionsbereiches in elektrische Datensignale und Verarbeitung der elektrischen Datensignale in einer Auswerte- und Steuereinheit 6 (Schritt 120), Durchleuchten des Bruteies 12 zur Ermittlung der Position der Luftkammer 13 (Schritt 130), Positionieren einer Bearbeitungseinrichtung 8 zur Erzeugung der Sollbruchstelle 30 in der Kalkschale 11 des Bruteies 12 und Bearbeiten des stumpfen Pols 14 zur Erzeugung der Öffnung 31 in der Kalkschale 11 der Bruteier 12 (Schritt 140), Entfernen des längs der Sollbruchstelle 30 realisierten Deckels 22 (Schritt 150), Fokussieren der Farbkamera 5 auf die zu erfassende Zielstruktur 26 im Bereich der inneren Membran 19 (Schritt 160), Messen des Raman-Spektrums 24 der Zielstruktur 26 (Embryo) gemäß Fig. 6 (Schritt 170), Verschließen der Öffnung 31 mit einem Verschlusselement 34 (Schritt 180) und Abführen der Bruteier 12 mittels einer Abführungseinrichtung 1b in Laufrichtung 25 der Transporteinrichtung 1 (Schritt 190).

Die Einbringung der Öffnung 31 stellt eine definierte, zielgerichtete Bearbeitung am Brutei 12 dar, wobei die Ausgangspositionierung mittels des Sensors 32 oder des Sensor-Arrays und die zur Öffnungeinbringung zugeordnete Endpositionierung des Bruteies 12, die zur Öffnungeinbringung mit der zugehörige Einstellpositionierung der Bearbeitungseinrichtung 8 übereinstimmt, mittels des Einsatzes von in der Auswerteeinheit und Steuereinheit 6 gespeicherten programmtechnischen Mitteln durchgeführt werden.

Die angegebene gezielte Bearbeitung der Kalkschale 11 des Bruteies 12 kann neben der laser-optischen Bearbeitung auch mittels mechanischer, chemischer oder Wasserstrahl-Werkzeuge durchgeführt werden.

Es kann ebenfalls vorteilhaft sein, dass die Bearbeitung bei Brut-Temperaturen durchgeführt werden kann. Dazu kann die Vorrichtung 10, z.B. ohne die Auswerte- und Steuereinheit 6, innerhalb eines bruttemperierten Gehäuses (nicht eingezeichnet) eingebracht sein.

Als Eingangsgröße zur Herbeiführung der Öffnung 31 der Kalkschale 11 und zur Lokalisierung der am stumpfen Pol 14 befindlichen Luftkammer 13, werden zunächst z.B. mittels Lasertriangulations- bzw. Streiflichtverfahren die Positionen und Geometrien der typischerweise mit dem spitzen Ende 15 nach unten gelagerten Bruteier 12 aufgenommen. Anschließend werden die z.B. auf Standardbruthorden 16 gelagerten Eier 12, vorzugsweise mittels weißen Lichts von unten, d.h. vom spitzen Ende 15 ausgehend, wie in Fig. 2 mittels der ersten Detektionseinrichtung 2 zur ersten Durchleuchtung gezeigt, durchleuchtet. Dazu werden entsprechende Lichtquellen 9 (z.B. Halogenlampen, LED oder Standard-Schierlampen) unterhalb der Bruthorde 16 angeordnet (Fig. 1 und Fig. 2).

Durch das transmittierende Licht wird die Geometrie der Luftkammer 13 am stumpfen Ende 14 des Bruteies 12 sichtbar und mit Hilfe einer Kamera 39 detektiert und digitalisiert und als 2D-Bildbereich 3 in der Auswerte- und Steuereinheit 6 verarbeitet. Die Geometrien der möglichen Luftkammern 13 können, wie in Fig. 2 angegeben, unterschiedlich groß und auch unterschiedlich geneigt zur Eilängsachse ausgebildet sein.

Der in Fig. 3 gezeigte Umriss der hieraus erhaltenen, in die Ebene 3 projizierten Fläche 29 (Volumenprojektion der Luftkammer 13) entspricht im Realfall meist einer Ellipse.

Unter Zuhilfenahme der ermittelten Oberflächendaten (Lasertriangulation) und durch Berechnung des Schnittpunktes m der Ellipsenhauptachsen A, B aus dem digitalisierten Kamerabild 33 können im Anschluss zentrisch zum Schnittpunkt m über der aufgespannten inneren Membran 19 eine definierte Berechnung der Sollbruchstelle 30 und damit des Kalkschalendeckels 22 und somit die Eröffnung der Kalkschale 11 erfolgen. Dafür kommen Materialbearbeitungslaser, wie z.B. CO₂-Laser, aber auch mechanische Verfahren, wie Fräsen, zum Einsatz.

Um eine ausreichend große Öffnung 31 für nachfolgende Untersuchungen zu schaffen, wird das Kalkschalenmaterial im ermittelten Bereich 21 als Kalkschalendeckel 22 gemäß Fig. 4 zirkulär, teilweise oder vollständig, vorzugsweise mit R < A/2 innerhalb der Sollbruchstelle 30 abgetragen und entfernt. Dies kann entweder mit Hilfe einer scannenden Laseroptik oder durch eine definierte Bewegung einer starren Laseroptik entlang der gewünschten Kontur 21 der Sollbruchstelle 30 erfolgen. Der so entstehende Deckel 22 kann anschließend mechanisch entfernt werden, wodurch ein freier Zugang zum Inneren der Luftkammer 13 und zu der an das Eiinnere angrenzenden inneren Membran 19 entsteht.

Durch erneute Anwendung der Lasertriangulation bzw. durch Verwendung eines einfachen optischen Abstandsmessers, kann gemäß Fig. 5 der Abstand a der inneren Membran 19 vom Eizenit 14 des stumpfen Endes bei der totalen Höhe b des Eies 12 exakt ermittelt werden. Damit ergibt sich die Möglichkeit für nachfolgende, zum Beispiel spektroskopische Verfahren, deren Fokusbereiche gemäß Fig. 5 exakt auf zu untersuchende, direkt an die innere Membran 19 angrenzende Zielstrukturen 26 auszurichten und geschlechtsspezifische Informationen aufzunehmen. Zur Lokalisierung der inneren Zielstrukturen 26 (z.B. embryonale Blutgefäße) werden die Bruteier 12 vorzugsweise mittels grünen Lichts der Lichtquelle 40 vom spitzen Ende 15 aus durchleuchtet und der freigelegte Bereich der inneren Membran 19 der Luftkammer 13 mittels der Farbkamera 5 und der Signalübergabe an die Auswerte- und Steuereinheit 6 aufgenommen. Diese kann durch eine aktive Nachfokussierung ebenfalls zur Abstandsermittlung der inneren Membran 19 mit dahinterliegenden Zielstrukturen 26 dienen.

Um eine optimierte Kontrastierung der zu untersuchenden, unter der inneren Membran 19 liegenden, spezifischen Zielstrukturen 26 (beispielsweise embryonale Blutgefäße) zu erhalten, kann die Lichtquelle 40 beispielsweise im grünen Spektralbereich zwischen 500 und 600nm zur Hämoglobin-Absorption eingesetzt werden.

Mit Hilfe der beschriebenen Vorrichtung 10 und des erfindungsgemäßen Verfahrens können, z.B. unter Anwendung der IR-Raman-Spektroskopie, wie in Fig. 6 für ein Einzel-Raman-Spektrum 24 gezeigt, charakteristische Raman-Spektren 24 embryonaler Blutgefäße 26 zur Bestimmung des Geschlechts des sich entwickelnden Vogelembryos 18 mittels der zur Raman-Spektren-Aufnahmeeinheit 38 gehörenden Messsonde 32 über den Strahlengang 23 aufgenommen werden.

Insbesondere können charakteristischen Raman-Spektren 24 mithilfe bekannter Verfahren der Datenanalyse ausgewertet werden, um das Geschlecht des sich entwickelnden Vogelembryos zu bestimmen.

Beispielsweise kann die bekannte Clusteranalyse zur Auswertung der charakteristischen Raman-Spektren 24 verwendet werden, um das Geschlecht des sich entwickelnden Vogelembryos zu bestimmen.

Bei der Clusteranalyse werden im Wesentlichen zwei Verfahren miteinander kombiniert, nämlich die Hauptkomponentenanalyse bzw. Principal Component Analysis (PCA) und K-Nächste-Nachbarn-Klassifikation.

Die PCA dient der Strukturierung und Vereinfachung der aufgenommenen Daten (z.B. charakteristisches Raman-Spektrum gemäß Figur 6). Dabei wird das gemessene Signal (Daten bzw. charakteristisches Raman-Spektrum) durch eine geringere Anzahl von Linearkombinationen dargestellt, als das Signal Messwerte hat. Dieser Schritt unterdrückt das Rauschen bei der Messung und macht die gemessenen Signale vergleichbarer. Nachdem das Signal in seine Hauptkomponenten zerlegt ist, ist es möglich, mit diesen Komponenten einen mathematischen Raum aufzustellen, d.h. die Parameter jeder Linearkombination einer Hauptkomponente entsprechen einem Punkt in einem zweidimensionalen Raum, bei dem die Achsen alle möglichen Kombinationen der Parameter einer möglichen Linearkombination sind. Es bildet sich somit eine Punktwolke. Diese Punktwolke wird im nächsten Schritt für die K-Nächste-Nachbarn-Klassifikation verwendet.

Bei den hier ausgewerteten Signalen (charakteristische Raman-Spektren) entspricht die K-Nächste-Nachbarn-Klassifikation k=1. Das stellt einen Spezialfall dar, und wird als Voronoi-Diagramm bezeichnet. Die gewonnene Punktwolke wird in Regionen unterteil, jeder Punkt bildet dabei das Zentrum einer Region. Auf dieses Konstrukt lassen sich verschiedene Metriken anwenden, z.B. die euklidische Distanz der Zentren zueinander oder die Flächengröße von bestimmten Regionen. Bestimmte Kombinationen von Zentren und Regionen sind spezifisch für ein Geschlecht des sich entwickelnden Vogelembryos und unterscheiden sich signifikant für männliche und weibliche Embryos.

Auch wenn an dieser Stelle beispielhaft die Clusteranalyse beschrieben wurde, können andere Datenanalyseverfahren zur Bestimmung des Geschlechts des sich entwickelnden Vogelembryos auf Basis der gewonnenen Signale (Daten bzw. charakteristische Raman-Spektren) verwendet werden. Die genannten bekannten Datenanalyseverfahren sind auch auf die mit anderen Analyseverfahren, wie Fluoreszenzspektroskopie oder weitere spektroskopische Verfahren anwendbar, insbesondere zur Auswertung charakteristischer Spektren.

Nach Aufnahme der charakteristischen Messdaten wird die am stumpfen Ende 14 des Bruteies 12 eingebrachte Öffnung 31 in der Kalkschale 11 wieder mittels eines Verschlusselements 34, z.B. mittels eines biokompatiblen Pflasters (z.B. mittels eines medizinischen Pflasters 3M DuraPore) oder einer Kappe mechanisch verschlossen. Die Eigenschaften des verwendeten Verschlussmaterials sind so gewählt, dass die Physiologie des Bruteis 12 nicht beeinflusst wird, und insbesondere aus der Luftkammer 13 ein überhöhter Verlust an Flüssigkeit durch Verdunsten vermieden wird.

Für die Einstellung einer Keimfreiheit von Anfang an ist eine Einrichtung 27 zur Desinfektion zumindest des Bereiches des stumpfen Pols 14 vorgesehen.

Während in den Ausführungsbeispielen die Verfahren und die Vorrichtung anhand von Messdaten der Ramanspektroskopie beispielhaft dargestellt wurden, ist eine äquivalente Anwendung durch die Verwendung von absoptionsspektroskopischen Verfahren, insbesondere der Fluoreszenzspektroskopie, in gleicher Weise möglich. Um eine Wiederholung der obigen Beschreibung zu vermeiden wird in Bezug auf die Anwendung von absoptionsspektroskopischen Verfahren, insbesondere der Fluoreszenzspektroskopie auf die Beschreibung der oben beschriebenen Ausführungsbeispiele verwiesen.

Während die vorliegende Erfindung hier unter Bezug auf ihre bevorzugten Ausführungsformen beschrieben und dargestellt wurde, ist für Fachleute auf dem Gebiet offensichtlich, dass verschiedene Modifikationen und Änderungen daran vorgenommen werden können, ohne den Schutzbereich der Erfindung zu verlassen. Auf diese Weise ist beabsichtigt, dass die vorliegende Erfindung die Modifikationen und Änderungen dieser Erfindung abdeckt, sofern sie in den Schutzbereich der beigefügten Patentansprüche und ihrer Äquivalente fallen.

Ferner können verschiedene Merkmale, die in Verbindung mit dem erfindungsgemäßen Verfahren beschrieben wurden, ebenfalls als Grundlage für Merkmale der erfindungsgemäßen Vorrichtung sein, und umgekehrt. Auch wenn bestimmte Merkmale in Kombination mit anderen Merkmalen beschrieben wurden, soll die vorliegende Erfindung nicht auf diese Kombinationen beschränkt sein. Es ist vielmehr eine, soweit technisch sinnvoll, beliebige Kombination von den beschriebenen Merkmalen möglich.

### Bezugszeichenliste

- 1: Transporteinrichtung
- 1a: Zuführungseinrichtung
- 1b: Abführungseinrichtung
- 2: Detektionseinrichtung zur Erfassung der Eiabmaße
- 3: 2D-Bildbereich
- 4: Detektionseinrichtung zur Erfassung der Abmaße der Luftkammer
- 5: Farbkamera mit Fokussierung auf eine Zielstruktur 26
- 6: Auswerte- und Steuereinheit
- 7: Verschließungseinrichtung
- 8: Bearbeitungseinrichtung
- 9: erste Lichtquelle zur ersten Durchleuchtung/erste Durchleuchtungseinrichtung
- 10: erfindungsgemäße Vorrichtung
- 11: Kalkschale
- 12: Brutei
- 13: Luftkammer innerhalb des Eies
- 14: stumpfer Pol
- 15: spitzer Pol
- 16: Einlage/Bruthorde
- 17: Bindungs-Grenze zwischen Luftkammer und innerer Membran
- 18: Embryo
- 19: innere Membran
- 20: äußere Membran
- 21: Bruchstellenprojektion
- 22: Kalkschalendeckel
- 23: Strahlengang zur Aufnahme eines Raman-Spektrums
- 24: Raman-Spektrum in Form einer Intensitäts-/Wellenzahl-Kurve
- 25: Laufrichtung des Transportbandes
- 26: Zielstruktur/Messbereich in der inneren Membran
- 27: Einrichtung zur Desinfektion
- 28: Einrichtung zur Abhebung des Deckels 22
- 29: Luftkammerprojektion
- 30: Sollbruchstelle
- 31: Öffnung in der Kalkschale
- 32: Messsonde
- 33: digitalisiertes Kamerabild
- 34: Verschlusselement
- 35: Positioniereinrichtung
- 36: Marker
- 37: Positionierungsbereich
- 38: Raman-Spektren-Aufnahmeeinheit/Einheit zur Aufnahme von Messdaten
- 39: Kamera für erste Durchleuchtungseinrichtung 9
- 40: zweite Lichtquelle zur zweiten Durchleuchtung/zweite Durchleuchtungseinrichtung

- A: erste kleine Ellipsenhauptachse
- B: zweite große Ellipsenhauptachse
- a: Abstand zwischen Messpunkt auf der inneren Membran und Eizenit des stumpfen Pols
- b: Höhe des Eies
- x: Koordinate
- y: Koordinate
- M: Zentrum der Eiprojektion
- m: Schnittpunkt der Luftkammerprojektion (Ellipse)
- R: Radius der Kreisprojektion mit R < A/2

- 100: Prozessschritt Zuführen
- 110: Prozessschritt Desinfizieren
- 120: Prozessschritt Erfassen
- 130: Prozessschritt Durchleuchten
- 140: Prozessschritt Öffnen
- 150: Prozessschritt Entfernen
- 160: Prozessschritt Fokussieren
- 170: Prozessschritt Messen
- 180: Prozessschritt Verschließen
- 190: Prozessschritt Abführen

## Patentansprüche

1. Verfahren zur Einbringung einer Öffnung (31) in die Kalkschale (11) eines bebrüteten Vogeleis (12) mit darin enthaltenem Embryo (18), im Bereich des stumpfen Pols (14) des bebrüteten Vogeleis (12), wobei sich innerhalb des Bereiches des stumpfen Pols (14) eine äußere Membran (20) und eine innere Membran (19) befinden, zwischen denen sich eine Luftkammer (13) befindet,
**dadurch gekennzeichnet, dass** das Verfahren die folgenden Schritte aufweist:
a) Lagern des bebrüteten Vogeleis (12) mit dem spitzen Pol (15) nach unten, wobei der Embryo (18) angrenzend an die innere Membran (19) anliegt;
b) Durchleuchten des bebrüteten Vogeleis (12) und Detektieren des durch das bebrütete Vogelei (12) hindurchgetretenen Lichtes zum Bestimmen der Position und der Geometrie der Luftkammer (13) am stumpfen Pol (14) des bebrüteten Vogeleis (12); und
c) Anschließendes Einbringen einer Öffnung (31) in die Kalkschale (11) am stumpfen Pol (14) des bebrüteten Vogeleis (12) oberhalb der aufgespannten inneren Membran (19) zur Luftkammer (13), um einen Zugang zur Luftkammer (13) zu erreichen.

2. Verfahren nach Anspruch 1, wobei das Verfahren ferner einen Schritt zum Bestimmen der Position und Geometrie der mit dem spitzen Pol (15) nach unten gelagerten bebrüteten Vogeleis (12) aufweist, wobei sich das bebrütete Vogelei (12) vorzugsweise auf einer vorgegebenen Bruthorde (16) befindet.

3. Verfahren nach Anspruch 1 oder 2, wobei der Schritt zum Bestimmen der Position und der Geometrie der Luftkammer (13) einen Schritt zum Bestimmen einer zweidimensionale Projektion (29) der Luftkammer (13) mit einem zentralen Punkt (m) aus dem detektierten, durch das bebrütete Vogelei hindurchgetretenen Licht aufweist, wobei die zweidimensionale Projektion (29) der Luftkammer (13) vorzugsweise eine im Wesentlichen elliptische Form mit dem Schnittpunkt (m) von sich schneidenden Ellipsenhauptachsen (A, B) als zentralen Punkt (m) aufweist.

4. Verfahren nach Anspruch 3, wobei der zentrale Punkt (m) als Mittelpunkt für das Einbringen der Öffnung (31) verwendet wird, wobei die Öffnung (31) vorzugsweise kreisförmig ist und einen Radius (R) besitzt der höchstens der Hälfte der kürzeren Ellipsenhauptachse entspricht.

5. Verfahren nach einem der Ansprüche 1 bis 4, wobei das Einbringen der Öffnung (31) einen Schritt zum Einbringen einer Sollbruchstelle (30) in die Kalkschale (11) und ferner einen Schritt zum Entfernen des durch die Sollbruchstelle (30) definierten Bereichs der Kalkschale (11) aufweist.

6. Verfahren nach einem der Ansprüche 1 bis 5, wobei das Verfahren nach dem Einbringen der Öffnung ferner einen weiteren Schritt zum Durchleuchten des bebrüteten Vogeleis (12) und einen Schritt zum Detektieren des durch das bebrütete Vogelei (12) hindurchgetretenen Lichtes umfasst, wobei vorzugsweise Licht im Spektralbereich von 500nm bis 600nm verwendet wird.

7. Verfahren nach Anspruch 6, wobei das Verfahren vor dem Detektieren des durch das bebrütete Vogelei (12) hindurchgetretenen Lichtes einen Schritt zum Ermitteln des Abstandes (a) der inneren Membran (19) vom Eizenit des stumpfen Pols (14) aus und einen Schritt zum Fokussieren des Lichts auf die innere Membran (19) unter Verwendung des Abstandes (a) aufweist.

8. Verfahren nach Anspruch 6 oder 7, wobei das Verfahren ferner einen Schritt zum Bestimmen der Position des Embryos (18) unter Verwendung des detektierten, durch das bebrütete Vogelei (12) hindurchgetretenen, Lichtes aufweist.

9. Verfahren nach einem der Ansprüche 1 bis 8, wobei das Verfahren ferner einen Schritt zum Bestimmen des Geschlechtes des Embryos (18) unter Verwendung von Absorptionssprektroskopie aufweist, vorzugsweise unter Verwendung von Ramanspektroskopie und/oder Fluoreszenzspektroskopie.

10. Verfahren nach einem der Ansprüche 1 bis 9, wobei das Verfahren nach dem Einbringen der Öffnung (31) ferner einen Schritt zum Verschließen der Öffnung, vorzugsweise mit einer semipermeablen Membran aus biokompatiblem Material, aufweist und ferner vorzugsweise einen Schritt zum Desinfizieren von mindestens dem stumpfen Pol (14) des bebrüteten Vogeleis (12) aufweist.

11. Vorrichtung (10) zur Einbringung einer Öffnung (31) in die Kalkschale (11) eines bebrüteten Vogeleis (12) mit darin enthaltenem Embryo (18), im Bereich des stumpfen Pols (14) des bebrüteten Vogeleis (12), wobei sich innerhalb des Bereiches des stumpfen Pols (14) eine äußere Membran (20) und eine innere Membran (19) befinden, zwischen denen sich eine Luftkammer (13) befindet, wobei die Vorrichtung vorzugsweise geeignet ist, das Verfahren gemäß den Ansprüchen 1 bis 10 durchzuführen,
wobei die Vorrichtung (10) aufweist:
eine Haltevorrichtung, die konfiguriert ist, das bebrütete Vogelei (12) mit dem spitzen Pol (15) nach unten zu lagern, wobei der Embryo (18) angrenzend an die innere Membran (19) anliegt;
**dadurch gekennzeichnet, dass** die Vorrichtung (10) ferner aufweist:
eine erste Detektionseinrichtung (4), die konfiguriert ist, die Position und Geometrie der Luftkammer (13) zu bestimmen, wobei die erste Detektionseinrichtung (4) eine erste Durchleuchtungseinrichtung (9), die konfiguriert ist, Licht durch das bebrütete Vogelei (12) zu senden, und einen ersten Detektor (39) aufweist, der konfiguriert ist, das durch das bebrütete Vogelei (12) transmittierte Licht aufzuzeichnen; und
eine Öffnungseinrichtung, die konfiguriert ist eine Öffnung (31) in die Kalkschale (11) am stumpfen Pol (15) des bebrüteten Vogeleis (12) oberhalb der aufgespannten inneren Membran (19) zur Luftkammer (13) einzubringen, um einen Zugang zu der Luftkammer (13) zu schaffen.

12. Vorrichtung nach Anspruch 11, wobei die erste Durchleuchtungseinrichtung (9) unterhalb des bebrüteten Vogeleis (12) angebracht ist und der erste Detektor (39) oberhalb des bebrüteten Vogeleis (12) und gegenüber der ersten Durchleuchtungseinrichtung (9) angebracht ist.

13. Vorrichtung nach Anspruch 11 oder 12, wobei eine zweite Detektionseinrichtung (2) konfiguriert ist, die Position und Geometrie des mit dem spitzen Pol (15) nach unten gelagerten bebrüteten Vogeleis (12) zu erfassen, wobei die zweite Detektionseinrichtung (2) vorzugsweise ein Sensor oder ein Sensor-Array ist, und vorzugsweise einen Abstandssensor oder einen Triangulationssensor aufweist.

14. Vorrichtung nach einem der Ansprüche 11 bis 13, wobei die Öffnungseinrichtung eine Bearbeitungseinrichtung (8) aufweist, die konfiguriert ist, eine Sollbruchstelle (30) in Form der Öffnung (31) einzubringen, wobei die Bearbeitungseinrichtung vorzugsweise eine laser-optische Einrichtung ist, die konfiguriert ist mit einem Laserstrahl eine Perforation der Kalkschale (11) durchzuführen, und wobei die Öffnungseinrichtung vorzugsweise eine Entfernungseinrichtung (28) aufweist, die konfiguriert ist den durch die Sollbruchstelle (30) definierten Bereich zu entfernen.

15. Vorrichtung nach einem der Ansprüche 11 bis 14, wobei die Vorrichtung ferner eine zweite Durchleuchtungseinrichtung (40) aufweist, die unterhalb des bebrüteten Vogeleis (12) angebracht ist und konfiguriert ist, Licht durch das bebrütete Vogelei (12) zu senden, wobei vorzugsweise Licht im Spektralbereich von 500nm bis 600nm verwendet wird, und wobei die Vorrichtung ferner einen zweiten Detektor (5) aufweist, der oberhalb des bebrüteten Vogeleis (12) und gegenüber der zweiten Durchleuchtungseinrichtung (40) angebracht ist, wobei der zweite Detektor (5) konfiguriert ist, das durch das bebrütete Vogelei (12) transmittierte Licht aufzuzeichnen, um die Position des Embryos (18) zu bestimmen.

16. Vorrichtung nach einem der Ansprüche 11 bis 15, wobei die Vorrichtung ferner eine Absorptionsspektroskopieeinheit (38) aufweist, die konfiguriert ist, das Geschlecht des Embryos (18) zu bestimmen, wobei die Absorptionsspektroskopieeinheit (38) vorzugsweise eine Raman-Spektroskopieeinheit und/oder eine Fluoreszenzspektroskopieeinheit ist.

17. Vorrichtung nach einem der Ansprüche 11 bis 16, wobei die Vorrichtung ferner eine Verschließungseinrichtung (7) zum Verschließen der Öffnung (31) aufweist, vorzugsweise unter Verwendung einer semipermeablen Membran aus biokompatiblem Material und wobei die Vorrichtung vorzugsweise eine Desinfektionseinrichtung (27) zum Desinfizieren mindestens des stumpfen Pols (14) des bebrüteten Vogeleis (12) aufweist.

## Claims

1. A method for creating an opening (31) in the calcified shell (11) of an incubated bird egg (12) containing an embryo (18), in the region of the blunt end (14) of the incubated bird egg (12), wherein within the region of the blunt end (14) there is an outer membrane (20) and an inner membrane (19) with an air cell (13) located therebetween,
**characterized in that** the method comprises the following steps:
a) storing the incubated bird egg (12) with its pointed end (15) facing downwards, wherein the embryo (18) adjoins the inner membrane (19);
b) candling the incubated bird egg (12) and detecting the light transmitted through the incubated egg (12) for detecting the position and the geometry of the air cell (13) at the blunt end (14) of the incubated bird egg (12); and
c) subsequently creating an opening (31) in the calcified shell (11) at the blunt end (14) of the incubated bird egg (12) above the taut inner membrane (19) to the air cell (13) in order to obtain an access to the air cell (13).

2. The method according to claim 1, wherein the method further comprises a step for detecting the position and geometry of the incubated bird egg (12) stored with its pointed end (15) facing downwards, wherein the incubated bird egg (12) is preferably located on a predetermined brooding tray (16).

3. The method according to claim 1 or 2, wherein the step for detecting the position and geometry of the air cell (13) comprises a step for determining a two-dimensional projection (29) of the air cell (13) with a central point (m) from the detected light transmitted through the incubated bird egg, wherein said two-dimensional projection (29) of the air cell (13) preferably comprises a substantially elliptic shape with the point of intersection (m) of intersecting major and minor axes (A, B) of the ellipse as a central point (m).

4. The method according to claim 3, wherein the central point (m) is used as the center for creating the opening (31), wherein the opening (31) is preferably circular and has a Radius (R) that at the most corresponds to half the minor axis of the ellipse.

5. The method according to any one of claims 1 to 4, wherein the creation of the opening (31) comprises a step for creating a predetermined breaking point (30) in the calcified shell (11) and further a step for removing the region of the calcified shell (11) defined by the predetermined breaking point (30).

6. The method according to any one of claims 1 to 5, wherein, after the opening has been created, the method additionally comprises a further step for candling the incubated bird egg (12), and a step for detecting the light transmitted through the incubated bird egg (12), wherein preferably light in the spectral range of 500 nm to 600 nm is used.

7. The method according to claim 6, wherein, prior to detecting the light transmitted through the incubated bird egg (12), the method comprises a step for determining the distance (a) of the inner membrane (19) starting from the vertex of the egg of the blunt end (14) and a step for focusing the light on the inner membrane (19) using the distance (a).

8. The method according to claim 6 or 7, wherein the method further comprises a step for detecting the position of the embryo (18) using the detected light transmitted through the incubated bird egg (12).

9. The method according to any one of claims 1 to 8, wherein the method further comprises a step for determining the sex of the embryo (18) using absorption spectroscopy, preferably using Raman spectroscopy and/or fluorescence spectroscopy.

10. The method according to any one of claims 1 to 9, wherein, after the opening (31) has been created, the method further comprises a step for closing the opening, preferably with a semipermeable membrane composed of a biocompatible material, and further preferably a step for disinfecting at least the blunt end (14) of the incubated bird egg (12).

11. An apparatus (10) for creating an opening (31) in the calcified shell (11) of an incubated bird egg (12) containing an embryo (18), in the region of the blunt end (14) of the incubated bird egg (12), wherein within the region of the blunt end (14) there is an outer membrane (20) and an inner membrane (19) with an air cell (13) located therebetween, wherein the apparatus is preferably configured to carry out the method according to claims 1 to 10,
wherein the apparatus (10) comprises:
a holder configured to store the incubated bird egg (12) with its pointed end (15) facing downwards, wherein the embryo (18) adjoins the inner membrane (19);
**characterized in that** the apparatus (10) further comprises:
a first detection device (4) configured to detect the position and geometry of the air cell (13), wherein the first detection device (4) comprises a first candling device (9) configured to send light through the incubated bird egg (12), and a first detector (39) configured to record the light transmitted through the incubated bird egg (12); and
an opening device configured to create an opening (31) in the calcified shell (11) at the blunt end (15) of the incubated bird egg (12) above the taut inner membrane (19) to the air cell (13) so as to obtain an access to the air cell (13).

12. The apparatus according to claim 11, wherein the first candling device (9) is arranged below the incubated bird egg (12) and the first detector (39) is arranged above the incubated bird egg (12) and opposite the first candling device (9).

13. The apparatus according to claim 11 or 12, wherein a second detection device (2) is configured to detect the position and geometry of the incubated bird egg (12) stored with its pointed end (15) facing downwards, wherein the second detection device (2) preferably is a sensor or a sensor array and preferably comprises a distance sensor or a triangulation sensor.

14. The apparatus according to any one of claims 11 to 13, wherein the opening device comprises a working device (8) configured to create a predetermined breaking point (30) in the form of the opening (31), wherein the working device preferably is a laser-optical device configured to perforate the calcified shell (11) with a laser beam, and wherein the opening device preferably comprises a removal device (28) configured to remove the region defined by the predetermined breaking point (30).

15. The apparatus according to any one of claims 11 to 14, wherein the apparatus further comprises a second candling device (40) arranged below the incubated bird egg (12) and configured to send light through the incubated bird egg (12), wherein preferably light in the spectral range of 500 nm to 600 nm is used, and wherein the apparatus further comprises a second detector (5) arranged above the incubated bird egg (12) and opposite the second candling device (40), wherein the second detector (5) is configured to record the light transmitted through the incubated bird egg (12) in order to detect the position of the embryo (18).

16. The apparatus according to any one of claims 11 to 15, wherein the apparatus further comprises an absorption spectroscopy unit (38) configured to determine the sex of the embryo (18), wherein the absorption spectroscopy unit (38) preferably is a Raman spectroscopy unit and/or a fluorescence spectroscopy unit.

17. The apparatus according to any one of claims 11 to 16, wherein the apparatus further comprises a closing device (7) for closing the opening (31), preferably using a semipermeable membrane composed of a biocompatible material, and wherein the apparatus preferably comprises a disinfection device (27) for disinfecting at least the blunt end (14) of the incubated bird egg (12).

## Revendications

1. Procédé pour la réalisation d'une ouverture (31) dans la coquille calcaire (11) d'un oeuf d'oiseau incubé (12) avec un embryon (18) contenu à l'intérieur, au niveau du pôle arrondi (14) de l'oeuf d'oiseau incubé (12), une membrane externe (20) et une membrane interne (19), entre lesquelles se trouve une chambre à air (13), se trouvant à l'intérieur de la zone du pôle arrondi (14),
**caractérisé en ce que** le procédé comprend les étapes suivantes :
a) pose de l'oeuf d'oiseau (12) avec le pôle pointu (15) vers le bas, l'embryon (18) s'appuyant à proximité de la membrane interne (19) ;
b) éclairage par transparence de l'oeuf d'oiseau incubé (12) et détection de la lumière ayant traversé l'oeuf d'oiseau incubé (12) pour déterminer la position et la géométrie de la chambre à air (13) au niveau du pôle arrondi (14) de l'oeuf d'oiseau incubé (12) ; et
c) réalisation d'une ouverture (31) dans coquille calcaire (11) au niveau du pôle arrondi (14) de l'oeuf d'oiseau incubé (12) au-dessus de la membrane interne tendue (19) vers la chambre à air (13), afin d'obtenir un accès à la chambre à air (13).

2. Procédé selon la revendication 1, le procédé comprenant en outre une étape de détermination de la position et de la géométrie de l'oeuf d'oiseau incubé (12) posé avec le pôle pointu (15) vers le bas, l'oeuf d'oiseau incubé (12) se trouvant de préférence sur un plateau d'incubation (16) prédéterminé.

3. Procédé selon la revendication 1 ou 2, l'étape de détermination de la position et de la géométrie de la chambre à air (13) comprenant une étape de détermination d'une projection bidimensionnelle (29) de la chambre à air (13) avec un point central (m) à partir de la lumière détectée ayant traversé l'oeuf d'oiseau incubé, la projection bidimensionnelle (29) de la chambre à air (13) comprenant de préférence une forme globalement elliptique avec le point d'intersection (m) des axes principaux de l'ellipse (A, B) en tant que point central (m).

4. Procédé selon la revendication 3, le point central (m) étant utilisé comme le centre pour la réalisation de l'ouverture (31), l'ouverture (31) étant de préférence circulaire et présentant un rayon (R) qui correspond au maximum à la moitié de l'axe principal le plus court de l'ellipse.

5. Procédé selon l'une des revendications 1 à 4, la réalisation de l'ouverture (31) comprenant une étape de réalisation d'un point de rupture prédéfini (30) dans la coquille calcaire (11) et en outre une étape d'élimination de la zone de la coquille calcaire (11) définie par le point de rupture prédéfini (30).

6. Procédé selon l'une des revendications 1 à 5, le procédé comprenant, après la réalisation de l'ouverture, en outre une étape supplémentaire d'éclairage par transparence de l'oeuf d'oiseau incubé (12) et une étape de détection de la lumière ayant traversé l'oeuf d'oiseau incubé (12), de préférence une lumière d'une plage spectrale de 500 nm à 600 nm étant utilisée.

7. Procédé selon la revendication 6, le procédé comprenant, avant la détection de la lumière ayant traversé l'oeuf d'oiseau incubé (12), une étape de détermination de la distance (a) entre la membrane interne (19) et le zénith du pôle arrondi (14) et une étape de focalisation de la lumière sur la membrane interne (19) à l'aide de la distance (a).

8. Procédé selon la revendication 6 ou 7, le procédé comprenant en outre une étape de détermination de la position de l'embryon (18) à l'aide de la lumière détectée ayant traversé l'oeuf d'oiseau incubé (12).

9. Procédé selon l'une des revendications 1 à 8, le procédé comprenant en outre une étape de détermination du sexe de l'embryon (18) à l'aide d'une spectroscopie par absorption, de préférence à l'aide d'une spectroscopie de Raman et/ou une spectroscopie par fluorescence.

10. Procédé selon l'une des revendications 1 à 9, le procédé comprenant, après la réalisation de l'ouverture (31), en outre une étape de fermeture de l'ouverture, de préférence avec une membrane semi-perméable en matériau biocompatible, et en outre de préférence une étape de désinfection d'au moins le pôle arrondi (14) de l'oeuf d'oiseau incubé (12).

11. Dispositif (10) pour la réalisation d'une ouverture (31) dans la coquille calcaire (11) d'un oeuf d'oiseau incubé (12) avec un embryon (18) contenu à l'intérieur, au niveau du pôle arrondi (14) de l'oeuf d'oiseau incubé (12), une membrane externe (20) et une membrane interne (19), entre lesquelles se trouve une chambre à air (13), se trouvant à l'intérieur de la zone du pôle arrondi (14), le dispositif étant de préférence conçu pour exécuter le procédé selon les revendications 1 à 10,
le dispositif (10) comprenant :
un dispositif de maintien qui est conçu pour poser l'oeuf d'oiseau incubé (12) avec le pôle pointu (15) vers le bas, l'embryon (18) s'appuyant à proximité de la membrane interne (19) ;
**caractérisé en ce que** le dispositif (10) comprend en outre :
un premier dispositif de détection (4), qui est conçu pour déterminer la position et la géométrie de la chambre à air (13), le premier dispositif de détection (4) comprenant un premier dispositif d'éclairage par transparence (9), qui est conçu pour émettre de la lumière à travers l'oeuf d'oiseau incubé (12), et comprend un premier détecteur (39) qui est conçu pour enregistrer la lumière transmise à travers l'oeuf d'oiseau incubé (12) ; et
un dispositif d'ouverture qui est conçu pour réaliser une ouverture (31) dans la coquille calcaire (11) au niveau du pôle arrondi (15) de l'oeuf d'oiseau incubé (12) au-dessus de la membrane interne tendue (19) vers la chambre à air (13), afin de créer un accès à la chambre à air (13).

12. Dispositif selon la revendication 11, le premier dispositif d'éclairage par transparence (9) étant monté en dessous de l'oeuf d'oiseau incubé (12) et le premier détecteur (39) étant monté au-dessus de l'oeuf d'oiseau incubé (12) et en face du premier dispositif d'éclairage par transparence (9).

13. Dispositif selon la revendication 11 ou 12, un deuxième dispositif de détection (2) étant conçu pour mesurer la position et la géométrie de l'oeuf d'oiseau incubé (12) posé avec le pôle pointu (15) vers le bas, le deuxième dispositif de détection (2) comprenant de préférence un capteur ou une matrice de capteurs et de préférence un capteur de distance ou un capteur à triangulation.

14. Dispositif selon l'une des revendications 11 à 13, le dispositif d'ouverture comprenant un dispositif de traitement (8) qui est conçu pour réaliser un point de rupture prédéfinie (30) ayant la forme de l'ouverture (31), le dispositif de traitement étant de préférence un dispositif optique au laser qui est conçu pour réaliser une perforation de la coquille calcaire (11) avec un rayon laser et le dispositif d'ouverture comprenant de préférence un dispositif d'élimination (28) qui est conçu pour éliminer la zone définie par le point de rupture prédéfinie (30).

15. Dispositif selon l'une des revendications 11 à 14, ce dispositif comprenant en outre un deuxième dispositif d'éclairage par transparence (40), qui est disposé en dessous de l'oeuf d'oiseau incubé (12) et qui est conçu pour émettre une lumière à travers l'oeuf d'oiseau incubé (12), de préférence une lumière dans la plage spectrale de 500 nm à 600 nm étant utilisée, et le dispositif comprenant en outre un deuxième détecteur (5) qui est disposé au-dessus de l'oeuf d'oiseau incubé (12) et en face du deuxième dispositif d'éclairage par transparence (40), le deuxième détecteur (5) étant conçu pour enregistrer la lumière transmise à travers l'oeuf d'oiseau incubé (12) afin de déterminer la position de l'embryon (18).

16. Dispositif selon l'une des revendications 11 à 15, ce dispositif comprenant en outre une unité de spectroscopie par absorption (38) qui est conçue pour déterminer le sexe de l'embryon (18), l'unité de spectroscopie par absorption (38) étant de préférence une unité de spectroscopie Raman et/ou une unité de spectroscopie par fluorescence.

17. Dispositif selon l'une des revendications 11 à 16, ce dispositif comprenant en outre un dispositif de fermeture (7) pour la fermeture de l'ouverture (31), de préférence à l'aide d'une membrane semi-perméable en matériau biocompatible et le dispositif comprenant de préférence un dispositif de désinfection (27) pour la désinfection d'au moins le pôle arrondi (14) de l'oeuf d'oiseau incubé (12).
